# EUROPEAN PATENT APPLICATION

(11) **EP 1 602 371 A2**
(43) Date of publication of application: **07.12.2005**
(21) Application number: 05019659.1
(22) Date of filing: 17.09.2002
(51) Int. Cl.: A61K 31/19, A61P 35/00

(54) **Valproic acid and derivatives for the sensitisation of human cancer cells to increase efficacy in a combination therapy**

(30) Priority: 18.09.2001 EP 01121722
(62) Divisional of application: 02777129.4
(71) Applicant: G2M Cancer Drugs AG, 60596 Frankfurt am Main (DE)
(72) Inventor: Groner, Bernd, 60322 Frankfurt am Main (DE); Heinzel, Thorsten, 55218 Ingelheim (DE); Hentsch, Bernd, 60322 Frankfurt am Main (DE); Wels, Winfried Stephan, 60599 Frankfurt (DE); Herrlich, Peter A., 76229 Karlsruhe (DE); Minucci, Saverio, 20090 Opera (IT); Pelicci, Pier Giuseppe, 20090 Opera (IT); Göttlicher, Martin, 80638 München (DE)
(74) Representative: Kalhammer, Georg

(57) **Abstract**

The present invention relates to the use of the drug valporic acid and derivatives thereof as inhibitors of enzymes having histone deacetylase activity for the manufacture of a medicament to sensitize human cancer cells for treatment efficacy in combination with clinically established anti-cancer therapeutic drugs. The invention also relates to the treatment of tumor metastasis and minimal residual disease.

## Description

The present invention relates to the use of the drug valproic acid and derivatives thereof for a sensitizing treatment of human cancers in combination with established therapeutic principles. The invention also relates to the use of those compounds for the treatment of tumor metastases and minimal residual disease. The invention includes the manufacture of a clinically used medicament for the treatment of human cancers.

Local remodeling of chromatin and dynamic changes in nucleosomal packaging of DNA are key steps in the regulation of gene expression and consequently affect proper cell function, differentiation and proliferation. One of the most important mechanisms determining the activity of target genes is the posttranslational modification of the N-terminal tails of core histones by acetylation and subsequent changes in chromatin structure (Davie, 1998, Curr Opin Genet Dev 8, 173-8; Kouzarides, 1999, Curr Opin Genet Dev 9, 40-8; Strahl and Allis, 2000, Nature 403, 41-4). Acetylation of lysine residues, predominantly in histones H3 and H4, is mediated by enzymes with histone acetyltransferase (HAT) activity. Conversely, acetyl groups are removed from ε-N-acetyl-lysine by histone deacetylases (HDACs). Both, HAT and HDAC activities can be recruited to target genes in complexes with sequence specific transcription factors and their cofactors. Nuclear receptors of the steroid/retinoid receptor superfamily such as retinoic acid receptor or thyroid hormone receptor are prototypical examples of transcription factors recruiting HAT and HDAC-associated cofactors depending on their status of activation by an appropriate ligand. In the absence of ligand these nuclear receptors interact with corepressors, e.g. N-CoR and SMRT. The corepressors form large protein complexes containing histone deacetylases and thereby inhibit transcription (Pazin and Kadonaga, 1997; Cell 89, 325-8). Upon ligand binding the corepressor complex dissociates and is replaced by coactivator proteins, e.g. SRC-1 and CBP, which exist in multiprotein complexes harboring histone acetyltransferase activity. The ligand-induced switch of nuclear receptors from repression to activation thus reflects the exchange of corepressor and coactivator complexes with antagonistic enzymatic activities (Glass and Rosenfeld, 2000, Genes Dev 14, 121-41). Intriguingly, many other transcription factors such as Mad-1, BCL-6, and ETO 4 (Pazin and Kadonaga, 1997, Cell 89, 325-8; Huynh and Bardwell, 1998, Oncogene 17, 2473-84; Wang, J. et al., 1998, Proc Natl Acad Sci USA 95, 10860-5) have also been shown to assemble HDAC-dependent transcriptional repressor complexes, indicating that this is a common mechanism of gene regulation.

Mammalian histone deacetylases can be divided into three subclasses (Cress and Seto, 2000, J Cell Physiol 184, 1-16; Gray and Ekstrom 2001, Exp Cell Res 262, 75-83). Class I enzymes are homologues of the yeast RPD3 protein and include the mammalian HDAC1, HDAC2, HDAC3 and HDAC8 enzymes with molecular masses ranging from 42 to 55 kDa. Class II histone deacetylases HDAC4, HDAC5, HDAC6 and HDAC7 are larger proteins (about 120 to 130 kDa) which are related to the yeast HDA1 protein. Recently, a third class of histone deacetylases with homology to the yeast SIR2 protein and several putative mammalian members has been identified (Imai et al., 2000, Nature 403, 795-800). Presently, it is still unclear to which extent these HDACs exert isoenzyme-specific or redundant functions. Further studies including gene deletion analysis are therefore required to elucidate the biological roles of each of these enzymes.

Histone deacetylases bind to many different proteins and usually exist in large complexes within the cell. Many of the associated proteins seem to be involved in either targeting HDACs to their substrates or to transcriptional repressors. For example, the Rb-associated proteins RbAP46 and RbAP48 are usually considered as integral parts of the HDAC enzyme complex responsible for the recognition of nucleosomal substrates (Taunton et al., 1996, Science 272, 408-11; Verreault et al., 1996, Cell 87, 95-104). The corepressors N-CoR, SMRT and Sin3 on the other hand are bridging factors required for the recruitment of HDACs to transcription factors (Pazin and Kadonaga, 1997, Cell 88, 737-40). Histone deacetylases are also components of the nucleosome remodeling and deacetylase (NuRD) complex which also contains RbAP46 and RbAP48, Mi-2 and MTA2 (Zhang, Y. et al., 1999, Genes Dev 13, 1924-35). Given the large number of HDAC isoenzymes and interacting proteins it is conceivable that complex composition could modulate substrate specificity and target HDACs even to non-histone proteins.

Inappropriate repression of genes required for cell differentiation has been linked to several forms of cancer and in particular to acute leukemia. In acute promyelocytic leukemia (APL) patients, RAR fusion proteins resulting from chromosomal translocations involve either the promyelocytic leukemia protein (PML) or the promyelocytic zinc finger protein (PLZF) (de The, 1996, Faseb J 10, 955-60). Both fusion proteins can interact with components of the corepressor complex. The addition of high doses of all-trans retinoic acid, however, dismisses the corepressor complex only from PML-RAR, but not from PLZF-RAR (Grignani et al., 1998, Nature 391, 815-8; Guidez et al., 1998, Blood 91, 2634-42; He et al., 1998, Nat Genet 18, 126-35; Lin et al., 1998, Nature 391, 811-4). These findings provide an explanation why PML-RAR APL patients usually achieve complete remission upon retinoic acid treatment whereas PLZF-RAR APL patients respond very poorly to this therapy. The hypothesis that corepressor-mediated aberrant repression may be causal for pathogenesis in APL is supported by the finding that inhibitors of corepressor-associated HDAC activity are capable of overcoming the differentiation block in cells containing the PLZF-RAR fusion protein.

In a frequent form of acute myeloid leukemia (AML), the translocation t(8;21) results in the AML1/ETO fusion protein, in which the transactivation domain of transcription factor AML1 is replaced by almost the entire ETO protein. The translocation partner ETO has been reported to interact with N-CoR, SMRT, mSin3 and HDACs (Lutterbach et al., 1998, Mol Cell Biol 18, 7176-84; Gelmetti et al., 1998, Mol Cell Biol 18, 7185-91; Wang et al., 1998, Proc Natl Acad Sci USA 95, 10860-5; Hildebrand et al., 2001, J Biol Chem 276, 9889-95). Thus, the fusion protein recruits corepressor complexes containing HDAC activity instead of coactivators. Recent reports indicate that the oncogenic potential and transcriptional repressor activity of the translocation product AML1/ETO requires oligomerization (Minucci et al., 2000, Mol Cell 5, 811-20). In non-Hodgkin's lymphoma, translocations and point mutations frequently lead to overexpression of the BCL-6 oncogene product which has been implicated in the control of B-cell proliferation. Since BCL-6 is a transcription factor which has been shown to interact with the corepressors N-CoR and SMRT, aberrant repression as in acute leukemias could also be involved in the pathogenesis of non-Hodgkin's lymphoma (Huynh and Bardwell, 1998, Oncogene 17, 2473-84).

Mutations in a nuclear hormone receptor have also been implicated as causal agents in the syndrome of Resistance to Thyroid Hormone (RTH), an endocrine human genetic disease characterized by a disruption in both, negative-feedback regulation and positive regulation by T₃. Diverse dominant negative mutations in the thyroid hormone receptor beta (TRβ) gene causing constitutive binding of corepressors and associated HDACs are the molecular basis of RTH (Yoh et al., 1997, Mol Endocrinol 11, 470-80; Yoh and Privalsky 2000, Mol Cell Endocrinol 159, 109-24).

Since pathogenesis in acute leukemia and non-Hodgkin's lymphoma is associated with the aberrant repression of genes required for cell differentiation it is plausible that this mechanism could also be relevant for many additional types of cancer including solid tumors. Currently, the molecular basis of many neoplasias is still largely unexplored. Due to the link between transcriptional repression and the recruitment of histone deacetylases, inhibitors of this enzymatic activity can be expected to reverse repression and to induce re-expression of differentiation inducing genes. Therefore, HDAC inhibitors are potentially promising candidate drugs for differentiation therapy of cancer and the treatment of certain endocrine diseases.

The clinical benefits of HDAC inhibition and their implications for re-differentiation therapy are currently being investigated in several locations. A PML-RAR patient who had experienced multiple relapses after treatment with retinoic acid and chemotherapy has been treated with the HDAC inhibitor phenylbutyrate, resulting in complete remission of the leukemia (Warrell et al., 1998, J Natl Cancer Inst 90, 1621-4). The result of this initial study suggests that high doses of HDAC inhibitors need not to be permanently sustained in order to achieve a clinical response. Phase II studies in cancer patients will serve as proof of principle for the effectiveness of HDAC inhibitors in therapy.

Recently, it was discovered that the antiepileptic drug valproic acid (VPA, 2-propylpentanoic acid) acts as an inhibitor of histone deacetylases (PCT/EP01/07704; Phiel et al., 2001, J Biol Chem, in press). This activity can be separated by appropriate modifications of the VPA molecule from the hitherto therapeutically exploited antiepileptic activity (PCT/EP01/07704).

Valproic acid has multiple biological activities which depend on different molecular mechanisms of action:
- VPA is an antiepileptic drug.
- VPA is teratogenic. When used as an antiepileptic drug during pregnancy VPA can induce birth defects (neural tube closure defects and other malformations) in a few percent of born children. In mice, VPA is teratogenic in the majority of mouse embryos when properly dosed.
- VPA activates a nuclear hormone receptor (PPARδ). Several additional transcription factors are also derepressed but some factors are not significantly derepressed (glucocorticoid receptor, PPARα).
- VPA occasionally causes hepatotoxicity, which may depend on poorly metabolized esters with coenzyme A.

The use of VPA derivatives allowed to determine that the different activities are mediated by different molecular mechanisms of action. Teratogenicity and antiepileptic activity follow different modes of action because compounds could be isolated which are either preferentially teratogenic or preferentially antiepileptic (Nau et al., 1991, Pharmacol. Toxicol. 69, 310-321). Activation of PPARδ was found to be strictly correlated with teratogenicity (Lampen et al., 1999, Toxicol. Appl. Pharmacol. 160, 238-249) suggesting that, both, PPARδ activation and teratogenicity require the same molecular activity of VPA. Also, differentiation of F9 cells strictly correlated with PPARδ activation and teratogenicity as suggested by Lampen et al., 1999, and documented by the analysis of differentiation markers (Werling et al., 2001, Mol. Pharmacol. 59, 1269-1276). It was shown, that PPARδ activation is caused by the HDAC inhibitory activity of VPA and its derivatives (PCT/EP01/07704). Furthermore it was shown that the established HDAC inhibitor TSA activates PPARδ and induces the same type of F9 cell differentiation as VPA. From these results we conclude that not only activation of PPARδ but also induction of F9 cell differentiation and teratogenicity of VPA or VPA derivatives are most likely caused by HDAC inhibition.

Antiepileptic and sedating activities follow different structure activity relationships and thus obviously depend on a primary VPA activity distinct from HDAC inhibition. The mechanism of hepatotoxicity is poorly understood and it is unknown whether it is associated with formation of the VPA-CoA ester. HDAC inhibition, however, appears not to require CoA ester formation.

Today, tumor therapies are known which consist of the combinatorial treatment of patients with more than one anti-tumor therapeutic reagent. Examples are the combined use of irradiation treatment together with chemotherapeutic and/or cytotoxic reagents and more recently the combination of irradiation treatment with immunological therapies such as the use of tumor cell specific therapeutic antibodies. However, the possibility to combine individual treatments with each other in order to identify such combinations which are more effective than the individual approaches alone, requires extensive pre-clinical and clinical testing. It is not possible to predict which combinations show an additive or even synergistic effect. Besides the aim to increase the therapeutic efficacy, another purpose is the potential decrease of the doses of the individual components in the resulting combinations in order to decrease unwanted or harmful side effects caused by higher doses of the individual components.

The present invention aims at providing a method and/or medicament which is useful for the treatment of human cancer.
To this end it was now surprisingly found that VPA has unexpected beneficial effects when used for the treatment of potentially many different types of human cancer in combination with a whole variety of other anti-tumor therapies which are individually based on strikingly different modes of action. Thus, the potential therapeutic use of VPA as a component of many anti-tumor drug combinations may not be limited to combinations with drugs having particular molecular mechanisms. This in fact may render VPA a drug to be combined with the majority of existing anti-tumor approaches. Here, the precise mode of action which is employed by VPA is not fully understood, but its differentiation inducing potential may be the basis to sensitize tumor cells for the activity of such a wide range of anti-tumor drugs. This surprisingly broad potential of VPA is expected to be based on its activity as an inhibitor of specific sets of enzymes having HDAC activity.

Therefore, one aspect of the present invention is the use of VPA and derivatives thereof for a combinatorial treatment of a variety of human cancers. The anti-tumoral activity of such combinatorial treatments compared to the use of each component alone can thus be increased and - if desired - the doses of the individual components of such combinatorial treatments may be lowered in order to decrease unwanted side effects related to individual drugs. The invention also concerns the use of VPA or a derivative thereof for the manufacture of a medicament for a combinatorial treatment of human cancer.

As used herein, the term "combinatorial treatment" refers to a treatment of an individual with at least two different therapeutic agents. According to the invention, the individual is treated with a compound of formula I which constitutes the first therapeutic agent. The second therapeutic agent may be any clinically established anti-cancer therapy, e.g. radiation therapy or administration of a chemotherapeutic drug. A combinatorial treatment may include a third or even further therapeutic agent. In accordance with the invention the compound of formula I and the second and optionally further therapeutic agent can be administered simultaneously, or the compound of formula I can be administered prior to or after the second therapeutic agent. Administration of the compound of formula I prior to the second therapeutic agent or simultaneous administration is preferred. Administration (simultaneously or at a different time) can be done systematically or topically as determined by the indication. In addition, when the second therapeutic agent is radiation therapy, the compound of formula I can be administered to a cancer patient pre- or post-radiation therapy to treat or ameliorate the effects of cancer. When the first and second therapeutic agent are applied at a different time, the time between the two treatments is shorter than 10 days.

The terms "combinatorial treatment", "combination therapy" and "combined treatment" are used interchangeably herein.

The derivatives of VPA are α-carbon branched carboxylic acids or carboxylic acid derivatives as described by formula I 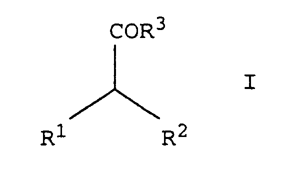 wherein R¹ and R² independently are a linear or branched, saturated or unsaturated aliphatic C₃₋₂₅ hydrocarbon chain which optionally comprises one or several heteroatoms and which may be substituted, R³ is hydroxyl, halogen, alkoxy or an optionally alkylated amino group.

Different R¹ and R² residues give rise to chiral compounds. Usually one of the stereoisomers has a stronger teratogenic effect than the other one (Nau et al., 1991, Pharmacol. Toxicol. 69, 310-321) and the more teratogenic isomer more efficiently activates PPARδ (Lampen et al, 1999). Therefore, this isomer can be expected to inhibit HDACs more strongly (PCT/EP01/07704). The present invention encompasses the racemic mixtures of the respective compounds and in particular the more active isomers.

The hydrocarbon chains R¹ and R² may comprise one or several heteroatoms (e.g. O, N, S) replacing carbon atoms in the hydrocarbon chain. This is due to the fact that structures very similar to that of carbon groups may be adopted by heteroatom groups when the heteroatoms have the same type of hybridization as a corresponding carbon group.

R¹ and R² may be substituted. Possible substituents include hydroxyl, amino, carboxylic and alkoxy groups as well as aryl and heterocyclic groups.

Preferably, R¹ and R² independently comprise 3 to 10, more preferably 4 to 10 or 5 to 10 carbon atoms. It is also preferred that R¹ and R² independently are saturated or comprise one double bond or one triple bond. In particular, one of the side chains (R¹) may preferably contain sp¹ hybridized carbon atoms in position 2 and 3 or heteroatoms which generate a similar structure. This side chain should comprise 3 carbon or heteroatoms but longer chains may also generate HDAC-inhibiting molecules. Also, inclusion of aromatic rings or heteroatoms in R² is considered to generate compounds with HDAC inhibitory activity because the catalytic site of the HDAC protein apparently accommodates a wide variety of binding molecules. With the observation that teratogenic VPA derivatives are HDAC inhibitors, also compounds which have previously been disregarded as suitable antiepileptic agents are considered as HDAC inhibitors (PCT/EP01/07704). In particular, but not exclusively, compounds having a propinyl residue as R¹ and residues of 7 or more carbons as R², are considered (Lampen et al, 1999).

Preferably, the group "COR³" is a carboxylic group. Also derivatization of the carboxylic group has to be considered for generating compounds with potential HDAC inhibitory activity. Such derivatives may be halides (e.g. chlorides), esters or amides. When R³ is alkoxy, the alkoxy group comprises 1 to 25, preferably 1-10 carbon atoms. When R³ is a mono- or di-alkylated amino group, the alkyl substituents comprise 1 to 25, preferably 1-10 carbon atoms.

According to the present invention also pharmaceutically acceptable salts of a compound of formula I can be used for combinatorial therapy of cancer. According to the present invention also substances can be used which are metabolized to a compound as defined in formula I in the human organism or which lead to the release of a compound as defined in formula I for example by ester hydrolysis.

In a particular embodiment, the invention concerns the use of an α-carbon branched carboxylic acid as described in formula I or of a pharmaceutically acceptable salt thereof as an inhibitor of an enzyme having histone deacetylase activity and its use in combinatorial therapy of cancer, wherein R¹ is a linear or branched, saturated or unsaturated, aliphatic C₅₋₂₅ hydrocarbon chain, R² independently is a linear or branched, saturated or unsaturated, aliphatic C₂₋₂₅ hydrocarbon chain, but not -CH₂-CH=CH₂, -CH₂-C≡CH or -CH₂-CH₂-CH₃, R¹ and R² are optionally substituted with hydroxyl, amino, carboxylic, alkoxy, aryl and/or heterocyclic groups, and R³ is hydroxyl.

In yet another embodiment the invention concerns the use of an α-carbon branched carboxylic acid as described in formula I or of a pharmaceutically acceptable salt thereof for the combinatorial therapy of cancer, wherein R¹ is a linear or branched, saturated or unsaturated, aliphatic C₃₋₂₅ hydrocarbon chain, and R² independently is a linear or branched, saturated or unsaturated, aliphatic C₃₋₂₅ hydrocarbon chain, R¹ or R² comprise one or several heteroatoms (e.g. O, N, S) replacing carbon atoms in the hydrocarbon chain, R¹ and R² are optionally substituted with hydroxyl, amino, carboxylic, alkoxy, aryl and/or heterocyclic groups, and R³ is hydroxyl.

In yet another embodiment of the invention R¹ and R² do not comprise an ester group (-COO-). R¹ and R² may be hydrocarbon chains comprising no heteroatoms O, N or S.

The compounds which are most preferably used according to the present invention are VPA and/or 4-yn VPA.

In one embodiment, the compound of formula I is used for the manufacture of a medicament to sensitize human cancer cells for treatment efficacy in combination therapy with clinically established anti-cancer therapeutic agents. Cancer cells are sensitized upon contact with a sensitizing agent when a lower dose of a given anti-cancer agent is required to achieve a certain anti-cancer effect compared with cancer cells which have not been contacted with said sensitizing agent. Anti-cancer effects may be reduction in tumor mass, inhibition of proliferation and/or cytotoxicity. Methods to determine anti-tumor effects are known to those skilled in the art. Example 1 shows, for instance, that a lower concentration of 5-FU is required to achieve a certain reduction in cellular biomass of colon cancer cells when used in combination with VPA.

According to the present invention, VPA or derivatives thereof are used for a combinatorial treatment of human cancer or for the manufacture of a medicament for a combinatorial treatment of human cancer. The combinatorial treatment may comprise known methods of anti-tumor therapy.

For those cancers which manifest themselves as solid tumors, the most efficient way of treatment is to surgically remove the tumor mass. For early stage tumors, which are completely resectable, drug therapy is infrequently used. At later stages however, the tumor has usually grown to such a size and/or has spread through the body to such an extent that resection is no longer a suitable treatment option. In these cases, drug therapy is used either to reduce the size of the tumor before resection, or to eliminate residual cancer cells (minimal residual disease) in the body after tumor resection.

Today, there are different classes of anti-cancer drug therapies including chemotherapeutic and cytotoxic reagents, differentiation-inducing reagents (e.g. retinoic acid, vitamin D, cytokines), hormonal therapy, immunological approaches and, more recently, the development of anti-angiogenic approaches. These methods may be used as second therapeutic agents in combination with treatment by VPA or a derivative thereof and are explained in more detail below.

### Chemotherapeutic and cytotoxic drugs:

Such drugs are used, usually in addition to standard surgical procedures, in an attempt to damage any cells that are actively growing and dividing. In most cases, there are more cancer cells going through the process of growing and dividing than normal cells are, so chemotherapeutics and cytotoxics have a more profound effect on cancer cells than on normal cells. Chemotherapeutic/cytotoxic drugs can be separated into distinct classes, including:
- alkylating agents
- cytotoxic antibiotics
- antimetabolites
- vinca alkaloids and etoposide
- others

Alkylating agents react with nucleophilic residues, such as the chemical entities on the nucleotide precursors for DNA production. They affect the process of cell division by alkylating these nucleotides and preventing their assembly into DNA.
Cytotoxic antibiotics act by directly inhibiting DNA or RNA synthesis and are effective throughout the cell cycle.
Antimetabolites interfere with cellular enzymes or natural metabolites that are involved in the process of cell division, thus disrupting the division of the cell.

Plant alkaloids and etoposides are agents derived from plants. They inhibit cell replication by preventing the assembly of the cell's components that are essential to cell division (e.g. Vinca alkaloids; Etoposide).
The group of compounds labelled 'Others' is made up primarily of taxanes (e.g. Paclitaxel, Taxol, Docetaxel, Taxotere) and metal complexes (e.g. cisPlatinum).

### Hormonal therapies:

The progression of some cancers, e.g. of the breast or prostate, depends on an excess or absence of hormones in the body. In these cases, hormonal therapies are used to boost or reduce hormone levels in the body with the aim to inhibit tumor growth in these organs.
There are five main classes of products in the hormonal therapies segment:
- progestogens
- anti-androgens
- anti-oestrogens
- lutenising hormone release hormone (LHRH) analogues
- aromatase inhibitors.
   Progestogens are used in the treatment of endometrial cancers, since these cancers occur in women that are exposed to high levels of oestrogen unopposed by progestogen.

Anti-androgens are used primarily for the treatment of prostate cancer, which is hormone dependent. They are used to decrease levels of testosterone, and thereby inhibit growth of the tumor.

Hormonal treatment of breast cancer involves reducing the level of oestrogen-dependent activation of oestrogen receptors in neoplastic breast cells. Anti-oestrogens act by binding to oestrogen receptors and prevent the recruitment of coactivators, thus inhibiting the oestrogen signal.

The LHRH analogues used in the treatment of prostate cancer act to decrease levels of testosterone and so decrease the growth of the tumor.

Finally, Aromatase inhibitors act by inhibiting the enzyme required for hormone synthesis. In post-menopausal women, the main source of oestrogen is through the conversion of androstenedione to estrone by aromatase.

Innovative therapies:
Until the 1990s, cytotoxics and hormonal therapies were the basis of drug treatment of cancer. However, recent developments have introduced additional categories in the innovative therapies segment, including:
   - gene therapies
   - immunotherapies
   - anti-angiogenic approaches (of lymphatic and blood vessels)

To date, there are no genetic therapies approved for clinical use in cancer patients, but many forms of gene therapy are undergoing preclinical or early clinical trials.

The body responds to cancer and how those responses assist the body to deal with cancer cells has been investigated intensively. Resulting anti-tumor approaches include immunotherapy with antibodies and reagents used in tumor vaccination approaches. The primary drugs in this therapy class are antibodies, alone or carrying e.g. toxins or chemotherapeutics/cytotoxics to cancer cells.

Last but not least, therapeutic anti-tumor approaches are currently under development which are based on the inhibition of tumor vascularization (anti-angiogenesis). The aim of this concept is to cut off the tumor from nutrition and oxygen supply provided by a newly built tumor vascular system.

The compound of formula I or derivatives thereof usually exhibit a HDAC inhibitory activity and frequently and unexpectedly cause a synergistic therapeutic effect upon combinatorial therapy with one or several other anti-cancer treatments which target mechanisms strikingly different from each other.

The compound of formula I is usually capable of sensitizing human cancer cells. Therefore, the compound of formula I is also termed the sensitizing agent of formula I herein. As a consequence, the dosage of the second therapeutic agent in a combination therapy can be significantly reduced to achieve an anti-tumor effect when used in combination with the sensitizing agent of formula I. The dosage of the second therapeutic agent preferably can be reduced by at least 25% compared to the dosage usually administered in clinical anti-cancer therapy. More preferably, the dosage can be reduced by at least 50%. The "dosage usually administered in clinical anti-cancer therapy" is defined herein as the amount of anti-cancer agent per sm (m²) or kg body weight (BW) of the patient per day (for references and dosing details see also S. Seeber and J. Schütte, Therapiekonzepte Onkologie, Springer-Verlag, 2. Auflage 1998, ISBN 3-540-58586-9).

Commonly used anti-cancer agents and daily dosages usually administered include but are not restricted to:

| | | |
|---|---|---|
| Antimetabolites | 1. Methotrexate: | 20-40 mg/m² i.v. |
| | | 4-6 mg/m² p.o. |
| | | 12 000 mg/m² high dose therapy |
| | 2. 6-Mercaptopurine: | 100 mg/ m² |
| | 3. 6-Thioguanine: | 1-2 x 80 mg/m² p.o. |
| | 4. Pentostatin | 4 mg/ m² i.v. |
| | 5. Fludarabinphosphate: | 25 mg/ m² i.v. |
| | 6. Cladribine: | 0.14 mg/kg BW i.v. |
| | 7. 5-Fluorouracil | 500-2600 mg/ m² i.v. |
| | 8. Capecitabine: | 1250 mg/ m² p.o. |
| | 9. Cytarabin: | 200 mg/ m² i.v. |
| | | 3000 mg/ m² i.v. high dose therapy |
| | 10. Gemcitabine: | 800-1250 mg/ m² i.v. |
| | 11. Hydroxyurea: | 800-4000 mg/ m² p.o. |
| Antibiotics | 12. Actinomycin D | 0.6 mg/ m² i.v. |
| | 13. Daunorubicin | 45-60 mg/ m² i.v. |
| | 14. Doxorubicin | 45-60 mg/ m² i.v. |
| | 15. Epirubicin | 60-80 mg/ m² i.v. |
| | 16. Idarubicin | 10-12 mg/ m² i.v. |
| | | 35-50 mg/ m² p.o. |
| | 17. Mitoxantron | 10-12 mg/ m² i.v. |
| | 18. Bleomycin | 10-15 mg/ m² i.v., i.m., s.c. |
| | 19.Mitomycin C | 10-20 mg/ m² i.v. |
| | 20. Irinotecan (CPT-11) | 350 mg/ m² i.v. |
| | 21. Topotecan | 1.5 mg/ m² i.v. |
| Alkylating agents | 22. Mustargen | 6 mg/ m² i.v. |
| | 23. Estramustinphosphate | 150-200 mg/ m² i.v. |
| | | 480-550 mg/ m² p.o. |
| | 24. Melphalan | 8-10 mg/ m² i.v. |
| | | 15 mg/ m² i.v. |
| | 25. Chlorambucil | 3-6 mg/ m² i.v. |
| | 26. Prednimustin | 40-100 mg/ m² p.o. |
| | 27. Cyclophosphamide | 750-1200 mg/ m² i.v. |
| | | 50-100 mg/ m² p.o. |
| | 28. Ifosfamid | 1500-2000 mg/ m² i.v. |
| | 29. Trofosfamid | 25-200 mg/ m² p.o. |
| | 30. Busulfan | 2-6 mg/ m² p.o. |
| | 31. Treosulfan | 5000-8000 mg/ m² i.v. |
| | | 750-1500 mg/ m² p.o. |
| | 32. Thiotepa | 12-16 mg/ m² i.v. |
| | 33. Carmustin (BCNU) | 100 mg/ m² i.v. |
| | 34. Lomustin (CCNU) | 100-130 mg/ m² p.o. |
| | 35. Nimustin (ACNU) | 90-100 mg/ m² i.v. |
| | 36. Dacarbazine (DTIC) | 100-375 mg/ m² i.v. |
| | 37. Procarbazine | 100 mg/ m² p.o. |
| | 38. Cisplatin | 20-120 mg/ m² i.v. |
| | 39. Carboplatin | 300-400 mg/ m² i.v. |
| Anti-mitotic agents | 40. Vincristin | 1.5-2 mg i.v. |
| | 41. Vinblastin | 4-8 mg/ m² i.v. |
| | 42. Vindesin | 2-3 mg/ m² i.v. |
| | 43. Etoposide (VP16) | 100-200 mg/ m² i.v. |
| | | 100 mg p.o. |
| | 44. Teniposide (VM26) | 20-30 mg/ m² i.v. |
| | 45. Paclitaxel (Taxol) | 175-250 mg/ m² i.v. |
| | 46. Docetaxel (Taxotere) | 100-150 mg/ m² i.v. |
| Hormones, Cytokines and Vitamins | 47. Interferon-α | 2-10 x 10⁶ IU/ m² |
| | 48. Prednison | 40-100 mg/ m² p.o. |
| | 49. Dexamethason | 8-24 mg p.o. |
| | 50. G-CSF | 5-20 µg/kg BW s.c. |
| | 51. *all*-trans Retinoic Acid | 45 mg/ m² |
| | 52. Interleukin-2 | 18 x 10⁶ IU/ m² |
| | 53. GM-CSF | 250 mg/ m² |
| | 54. erythropoietin | 150 IU/kg tiw |
| Other | 55. Radiation | 20-60 Gy |

The daily dosages of the second therapeutic anti-cancer agents described above can be significantly reduced in a combinatorial treatment with the sensitizing agent of formula I, compared to their usual dosages when administered alone or with other therapeutic principles. The following daily dosages may be used in the combinatorial treatment according to the invention:

| | | |
|---|---|---|
| Antimetabolites | 1. Methotrexate: | 10-30 mg/ m² i.v. |
| | | 2-4 mg/ m² p.o. |
| | | 6 000-8 000 mg/ m² high dose therapy |
| | 2. 6-Mercaptopurine: | 50-75 mg/ m² |
| | 3. 6-Thioguanine: | 1-2 x 40-60 mg/ m² p.o. |
| | 4. Pentostatin | 2-3 mg/ m² i.v. |
| | 5. Fludarabinphosphate: | 12-18 mg/ m² i.v. |
| | 6. Cladribine: | 0.7-11 mg/kg BW i.v. |
| | 7. 5-Fluorouracil | 250-1800 mg/ m² i.v. |
| | 8. Capecitabine: | 700-1000 mg/ m² p.o. |
| | 9. Cytarabin: | 100-150 mg/ m² i.v. |
| | | 1500-2200 mg/ m² i.v. high dose therapy |
| | 10. Gemcitabine: | 400-825 mg/ m² i.v. |
| | 11. Hydroxyurea: | 400-3000 mg/ m² p.o. |
| Antibiotics | 12. Actinomycin D | 0.3-0.45 mg/ m² i.v. |
| | 13. Daunorubicin | 20-45 mg/ m² i.v. |
| | 14. Doxorubicin | 20-45 mg/ m² i.v. |
| | 15. Epirubicin | 30-60 mg/ m² i.v. |
| | 16. Idarubicin | 5-9 mg/ m² i.v. |
| | | 18-38 mg/ m² p.o. |
| | 17. Mitoxantron | 5-9 mg/ m² i.v. |
| | 18. Bleomycin | 5-12 mg/ m² i.v., i.m., s.c. |
| | 19. Mitomycin C | 5-15 mg/ m² i.v. |
| | 20. Irinotecan (CPT-11) | 175-290 mg/ m² i.v. |
| | 21. Topotecan | 0.7-1.2 mg/ m² i.v. |
| Alkylating agents | 22. Mustargen | 3-4.5 mg/ m² i.v. |
| | 23. Estramustinphosphate | 75-150 mg/ m² i.v. |
| | | 240-400 mg/ m² p.o. |
| | 24. Melphalan | 4-7.5 mg/ m² i.v. |
| | | 7-12 mg/ m² i.v. |
| | 25. Chlorambucil | 1.5-4.5 mg/ m² i.v. |
| | 26. Prednimustin | 20-75 mg/ m² p.o. |
| | 27. Cyclophosphamide | 375-900 mg/ m² i.v. |
| | | 25-75 mg/ m² p.o. |
| | 28. Ifosfamid | 750-1500 mg/ m² i.v. |
| | 29. Trofosfamid | 12-150 mg/ m² p.o. |
| | 30. Busulfan | 1-4.5 mg/ m² p.o. |
| | 31. Treosulfan | 2500-6000 mg/ m² i.v. |
| | | 375-1200 mg/ m² p.o. |
| | 32. Thiotepa | 6-12 mg/ m² i.v. |
| | 33. Carmustin (BCNU) | 50-75 mg/ m² i.v. |
| | 34. Lomustin (CCNU) | 50-95 mg/ m² p.o. |
| | 35. Nimustin (ACNU) | 45-750 mg/ m² i.v. |
| | 36. Dacarbazine (DTIC) | 50-280 mg/ m² i.v. |
| | 37. Procarbazine | 50-75 mg/ m² p.o. |
| | 38. Cisplatin | 10-90 mg/ m² i.v. |
| | 39. Carboplatin | 150-300 mg/ m² i.v. |
| Anti-mitotic agents | 40. Vincristin | 0.75-1.5 mg i.v. |
| | 41. Vinblastin | 2-6 mg/ m² i.v. |
| | 42. Vindesin | 1-2.2 mg/ m² i.v. |
| | 43. Etoposide (VP16) | 50-150 mg/ m² i.v. |
| | | 50-75 mg p.o. |
| | 44. Teniposide (VM26) | 10-22 mg/ m² i.v. |
| | 45. Paclitaxel (Taxol) | 80-180 mg/ m² i.v. |
| | 46. Docetaxel (Taxotere) | 50-120 mg/ m² i.v. |
| Hormones, Cytokines and Vitamins | 47. Interferon-α | 1-5 x 10⁶ IU/m² |
| | 48. Prednison | 20-75 mg/m² p.o. |
| | 49. Dexamethason | 4-18 mg p.o. |
| | 50. G-CSF | 2.5-15 µg/kg BW s.c. |
| | 51. all-trans Retinoic Acid | 22-35 mg/ m² |
| | 52. Interleukin-2 | 9-14 x 10⁶ IU/ m² |
| | 53. GM-CSF | 125-180 mg/ m² |
| | 54. erythropoietin | 75-120 IU/kg tiw |
| Other | 55. Radiation | 10-45 Gy |

A further aspect of the invention is a pharmaceutical kit comprising as a first therapeutic agent a compound of formula I and as a second therapeutic agent an anti-cancer agent, wherein the anti-cancer agent is provided in a form suitable for administration in a dosage which is reduced by at least 25% compared to the dosage usually administered in clinical anti-cancer therapy. Preferably, the dosage is reduced by at least 50%. The components of the kit may be placed in a container, they may also be packaged in a form suitable for separate administration of the respective components.

Yet another aspect of the invention is a method for reducing the dosage of an anti-cancer agent comprising administering to a cancer patient an amount of a compound of formula I or a pharmaceutically acceptable salt thereof effective to sensitize cancer cells in the patient, wherein formula I has the same meaning as defined supra.

The invention further relates to a method of treating cancer in a patient which comprises administering to the patient an amount of a compound of formula I or a pharmaceutically acceptable salt thereof effective to sensitize the cancer cells in the patient to an anti-cancer agent and a therapeutically effective amount of the anti-cancer agent, wherein formula I has the same meaning as defined supra.

The invention further relates to a method of enhancing the therapeutic activity of an anti-cancer agent which comprises administering to a patient an amount of a compound of formula I or a pharmaceutically acceptable salt thereof effective to sensitize cancer cells in the patient to the anti-cancer agent, wherein formula I has the same meaning as defined supra.

The compounds of formula I may be useful for inhibiting mammalian (for use of cell lines in in vitro assays and animal models systems) and in particular human (in vivo and in vitro) histone deacetylases HDAC 1-3 and 8 (class I), HDAC 4-7 (class II), as well as a recently identified new class of histone deacetylases with homology to the yeast SIR2 protein including several putative mammalian members (Imai et al., 2000, Nature 403, 795-800) and for the use in cancer treatment in combination with other cancer therapies. In one embodiment the compound of formula I inhibits only a subset of HDACs.

Yet another aspect of the invention is the use of a compound of formula I for the manufacture of a medicament for the combinatorial treatment of a disease in which the induction of hyperacetylation of histones has a beneficial effect, e.g. resulting in differentiation and/or apoptosis of a patient's tumor cells and thus causing a clinical improvement of the patient's condition. Examples of such diseases are skin cancer, estrogen receptor-dependent and independent breast cancer, ovarian cancer, prostate cancer, renal cancer, colon and colorectal cancer, pancreatic cancer, head and neck cancer, small cell and non-small cell lung carcinoma. The induction of hyperacetylation may also be beneficial by reverting inappropriate gene expression in diseases based on aberrant recruitment of histone deacetylase activity such as thyroid resistance syndrome. The combinatorial treatment of the present invention is particularly useful for treating minimal residual tumor disease or tumor metastases.

The invention encompasses also the use of compounds which are metabolized in patients to a compound of formula I. The embodiments described in this invention apply to such compounds as well.

The compounds and salts thereof can be formulated as pharmaceutical compositions (e.g. powders, granules, tablets, pills, capsules, injections, solutions, foams, enemas and the like) comprising at least one such compound alone or in admixture with pharmaceutically acceptable carriers, excipients and/or diluents. The pharmaceutical compositions can be formulated in accordance with a conventional method. Specific dose levels for any particular patient will be employed depending upon a variety of factors including the activity of specific compounds employed, the age, body weight, general health, sex, diet, time of administration, route of administration, rate of excretion, drug combination, and the severity of the particular disease undergoing therapy. The sensitizing agent of formula I will preferably be administered in an appropriate amount, for example, selected from the range of about 10 mg/kg to 100 mg/kg body weight a day orally or intravenously. The dose levels are not specifically restricted as long as serum levels of 0.05 mM to 3 mM, preferably of about 0.4 mM to 1.2 mM are achieved.

Another aspect of the invention is a method for the identification of substances being useful for combinatorial cancer therapy which comprises providing a derivative of valproic acid, determining its histone deacetylase inhibitory activity, determining its efficiency in combinatorial cancer therapy and selecting the substance if the substance has histone deacetylase inhibitory activity and an efficiency in combinatorial cancer therapy which is significantly higher than that of the respective treatments alone.

Valproic acid can serve as a lead substance for the identification of other compounds exhibiting histone deacetylase inhibitory activity. Thereby compounds may be selected which show increased HDAC inhibitory activity at lower doses and serum levels and have decreased effects on the central nervous system such as sedating activity. Another parameter that may be optimized is the appearance of the hepatotoxic effect. Compounds may be selected which show reduced liver toxicity. The derivatives may be provided by synthesizing compounds which comprise additional and/or modified substituents. The HDAC inhibitory activity may be determined by a state-of-the-art technology such as transcription repression assay, a Western Blot which detects acetylation of histone H3 and/or histone H4, or by an enzymatic assay. Another parameter that may be optimized is the use of derivatives of VPA in combinatorial cancer therapy.

The transcriptional assay for repressor activity exploits activation and derepression of a Gal4-dependent reporter gene. This assay can be performed either by transient transfection of mammalian cell lines (e.g. HeLa, 293T, CV-1) or with specifically constructed permanent cell lines. Transcription factors such as thyroid hormone receptor, PPARδ, retinoic acid receptor, N-CoR and AML/ETO repress transcription when they bind to a promoter containing UAS elements as fusion proteins with the heterologous DNA-binding domain of the yeast Gal4 protein. In the absence of the Gal4-fusion protein the reporter gene has a high basal transcriptional activity due to the presence of binding sites for other transcription factors in the thymidine kinase promoter. The Gal4 fusion proteins repress this activity by up to 140-fold. HDAC inhibitors induce relief of this repression which can be detected as an increase in reporter gene activity (e.g. by luciferase assay).

Histone deacetylase inhibitors induce the accumulation of N-terminally hyperacetylated histones H3 and H4. These acetylated histones can be detected by Western blot analysis of whole cell extracts or of histone preparations from histone deacetylase inhibitor-treated cells using antibodies specific for the acetylated N-terminal lysine residues of histones H3 and H4.

The enzymatic assay for HDAC activity records the release of ³H-labeled acetic acid from hyperacetylated substrates. Sources of HDAC activity can be co-immunoprecipitates with antibodies directed against HDACs or N-CoR (or other repressors known to recruit HDACs) or crude cell extracts containing histone deacetylases (e.g. HeLa, 293T, F 9). Substrates may be either chemically ³H-acetylated peptides corresponding to the N-termini of histones H3 or H4 or histone proteins isolated from metabolically labelled cells which were treated with HDAC inhibitors. After extraction with ethyl acetate the release of ³H-labeled acetic acid is detected by liquid scintillation counting.

Yet another aspect of the invention is a method for profiling of the HDAC isoenzyme specificity of a compound as defined in formula I. For that purpose HDACs are either immune precipitated with HDAC isoform-specific antibodies, with antibodies directed against corepressor complexes, or with specific antibodies against recombinant HDACs overexpressed in transgenic cells. The method involves determination of individual HDACs present in these immune precipitates by Western blot analysis. Radiolabeled VPA or compounds according to formula I are bound to the immune precipitates. The amount of bound compound is determined through measurement of bound radioactivity after appropriate washing steps. A variation of this aspect involves binding of one labeled HDAC inhibitor such as VPA, TSA or trapoxin and competition of binding by a compound according to formula I. Another variation of the method involves the use of alternate labeling and/or detection procedures. It is preferred that compounds are selected which specifically inhibit only a subset of HDACs. The HDAC inhibition assay using chemically ³H-acetylated peptides as described above may also be used for the determination of HDAC inhibitory specificities.

A particular aspect of the invention is the use of VPA or derivatives thereof as described above, in combination with established therapeutic cancer treatments to define genes which are regulated by this combinatorial treatment in cells such as primary human or rodent cells, leukemic cells, other cancer cells or tumor cell lines. The invention thus concerns a method which comprises the steps of providing two populations of cells which are substantially identical, contacting the first population with VPA or a derivative thereof, subjecting the first population to treatment with one or several other methods of anti-tumor therapy, and detecting genes or gene products which are expressed in the first population which had been contacted with VPA or a derivative thereof and were subjected to treatment with one or several other methods of anti-tumor therapy at a level significantly higher than in the second population which had not been contacted with VPA or a derivative thereof. The step of contacting the first population with VPA or a derivative thereof and the step of subjecting the first population to treatment with one or several other methods of anti-tumor therapy may be carried out in any order or simultaneously.

Methods to define or identify such genes that are regulated by combinatorial treatment include established technologies for screening large arrays of cDNAs, expressed sequence tags or so-called unigene collections. Also the use of subtractive hybridization techniques is suitable to define genes which are regulated by such combinatorial treatments. The use of these methods to identify potential targets for drug development downstream of VPA-mediated HDAC-inhibition in combination with other drug mechanisms, and furthermore the use of these methods to define diagnostic means in order to facilitate the therapeutic treatment of patients with suitable compounds and combinations of treatments is part of this invention. Considering the low general toxicity of VPA in the organism compared to other HDAC-inhibitors it is a specific aspect of this invention to use VPA or derivatives thereof in combination with established cancer therapeutics for defining target genes which are selectively regulated or not regulated by these combinations, particularly also in comparison to the use of other HDAC inhibitors such as trapoxin or trichostatin A.

In a particular embodiment, the method for the identification of genes regulated by combinatorial treatment comprises the use of nucleic acid technology, preferably of hybridization or polymerase chain reaction for detection. Other types of nucleic acid technology, however, may be employed. In another embodiment the method comprises the use of specific antibodies against differentially regulated proteins for detection.

According to the present invention the expression level of a gene which has been identified according to the method for the identification of genes regulated by combinatorial treatment may be determined outside of the human or animal body for the diagnosis of tumors.

The present invention also concerns a diagnostic method to identify tumors comprising the step of testing in vitro whether a tumor is responsive to treatment with combinations of VPA or derivatives thereof and established tumor therapeutics. The method preferably comprises the method for the identification of genes regulated by these treatments. In a particular embodiment, the diagnostic method comprises the use of nucleic acid technology, preferably of hybridization or polymerase chain reaction for detection. Other types of nucleic acid technology, however, may be employed. In another embodiment the method comprises the use of specific antibodies against differentially regulated proteins for detection. For this purpose proteins encoded by the genes showing deregulation of their expression upon combinatorial treatment using VPA and derivatives thereof would be expressed e.g. in recombinant expression systems and antibodies directed against these proteins would be generated. Subsequently such antibodies could be used (or patterns of antibodies) to characterize the status of a tumor or tumor cells for diagnostic and/or prognostic reasons.

In general the present invention provides novel possibilities to treat various cancer diseases. Applicant found that the HDAC inhibitory and cellular differentiation-inducing activity of VPA and VPA-derivatives can be used successfully in combination with well established and clinically used therapeutic drugs for the treatment of tumor cells of different origins. Such VPA and derivatives thereof based combinatorial treatment is considered to generate superior therapeutic success in human tumor patients than the corresponding therapeutic drugs used on their own. It is an object of the present invention to provide combinatorial therapeutic approaches using VPA and derivatives for the treatment of cancer. Such combinatorial treatments could result in a decrease of the therapeutic doses of e.g. chemotherapeutic reagents required and could thus limit the currently observed, partly very serious side effects of frequently used therapies.

Aspects of the present invention are the combination of VPA or derivatives thereof with therapeutic principles currently in clinical use or in clinical development, such as
- Chemotherapeutic or cytotoxic drugs (e.g. 5-FU, taxol, cisPlatinum, camptothecin, gemcitabine, adriamicine, irinothecan)
- differentiation inducing drugs (e.g. vitamin D, retinoic acid, cytokines such as II-3, II-6, SCF, G-CSF, GM-CSF)
- Radiation therapy (e.g. x-rays or gamma rays)
- immunological approaches (antibody therapy, vaccination)
- combined immunotherapeutic/cytotoxic approaches (e.g. antibodies conjugated with cytotoxic components)
- anti-angiogenesis approaches.

The following compounds are not preferred as second therapeutic agent: TNFα, butyric acid, a butyric acid salt, a butyric acid derivative, IL-2, α-mercaptopropionylglycine, 9-aminocamptothecin, BCNU, Cytarabine, Teniposide, Vincristine, Cisplatin and/or Doxorubicin.

After tumor therapy often residual tumor cells remain in the patients' bodies. This condition is known as minimal residual disease. These tumor cells can give rise to secondary tumors even years after the primary tumor has been removed. Therefore, one major task of a successful tumor therapy must be the eradication of such residual tumor cells.
Thus, another aspect of the invention is the use of VPA and derivatives thereof for the inhibition of tumor metastasis and therefore the extinction of minimal residual disease.

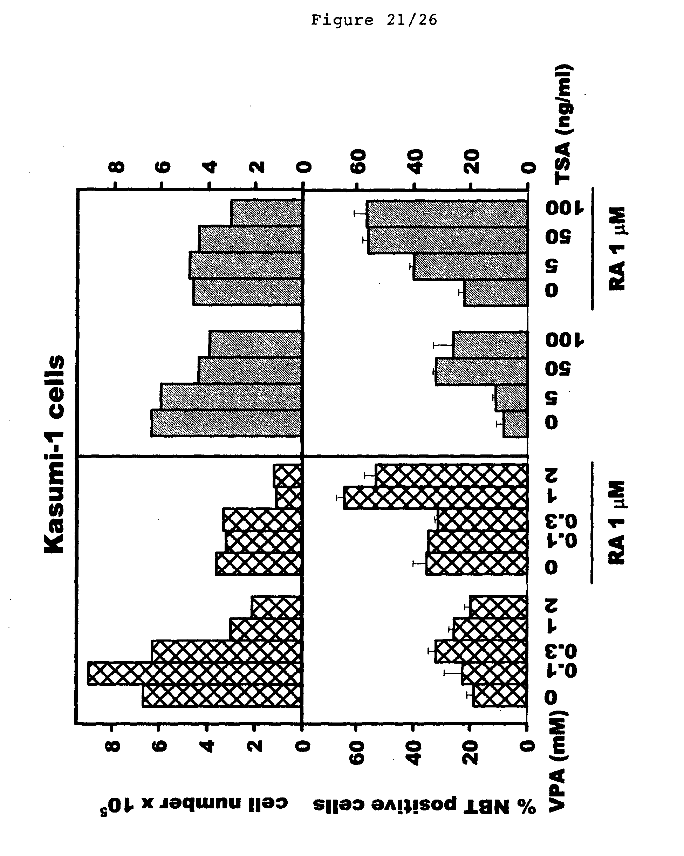

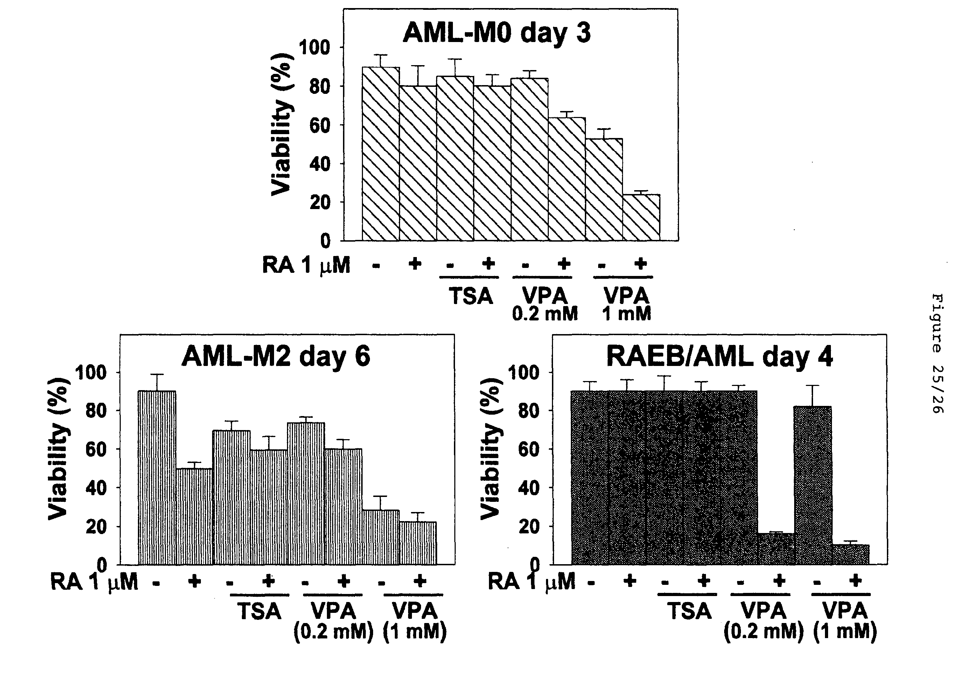

We tested the effect of established anti-tumor therapeutic principles in combination with VPA on human tumor cells of various origins. Surprisingly we regularly found that the combination of VPA with such established therapeutic principles had a synergistic anti-tumor effect compared to the effects seen with VPA alone or the tested established treatments alone. These frequently observed enhancements were unexpected, in particular since VPA caused its often synergistic effects together with fundamentally different therapeutic approaches, such as chemotherapy, different antibody therapies, irradiation treatment, differentiation inducing drugs or anti-angiogenic approaches. These approaches all target mechanisms which differ strikingly from each other. It could not be expected that one individual drug (VPA) would be able to enhance the therapeutic activity of such a wide and heterogeneous range of mechanistic anti-tumor approaches.
The most likely basis for this therapeutic success of VPA is its activity as a novel inhibitor of enzymes having HDAC activity. However, since e.g. TSA does not display this synergistic activities (see e.g. Figure 21 and 25) to the same extent VPA does, it appears that the fine tuned mechanistic targeting achieved by VPA appears to be superior to other HDAC inhibitors.
In addition, VPA may be employed for the inhibition of tumor metastases formation and thus for the treatment of minimal residual disease. This was successfully tested by using in vivo models of renal and breast carcinomas.

**Figure 1** shows the synergistic reduction in total cellular biomass of colon cancer cells by VPA in combination with the chemotherapeutic/cytotoxic drug 5-Fluorouracil, 5-FU (Example 1).

**Figure 2** shows the synergistic reduction in total cellular biomass of DU-145 prostate cancer cells by VPA in combination with the chemotherapeutic/cytotoxic drug cisPlatinum (Figure 2A-B) and the synergistic reduction in total cellular biomass of DU-145 cells achieved by the treatment with racemic 2-n-Propyl-4-pentynoic acid (4-yn VPA), a derivative of VPA (Figure 2C-D), in combination with the chemotherapeutic/cytotoxic drug cisPlatinum (Example 2).

**Figure 3** shows the at least additive reduction in total cellular biomass of breast cancer cells by VPA in combination with the chemotherapeutic/cytotoxic drug Paclitaxel (Example 3).

**Figure 4** shows the at least additive reduction in total cellular biomass of lung cancer cells by VPA in combination with the chemotherapeutic/cytotoxic drug Gemcitabine (Example 4).

**Figure 5** shows the at least additive reduction in total cellular biomass of colon cancer cells by VPA in combination with the chemotherapeutic/cytotoxic drug Camptothecine (Figure 5A-B) and the at least additive reduction in total cellular biomass of PC-3 prostate cancer cells achieved by the treatment with racemic 2-n-Propyl-4-pentynoic acid (4-yn VPA), a derivative of VPA (Figure 5C-D), in combination with the chemotherapeutic/cytotoxic drug Camptothecin (Example 5).

**Figure 6** displays the synergistic reduction in cellular viability of valproic acid and an immunotherapeutic approach using monoclonal antibodies (Example 6).
SKBR3 and MDA-MB453 breast carcinoma cells (A) or MDA-MB468 breast carcinoma cells and A431 squamous cell carcinoma cells (B) were incubated with 3 mM valproic acid (VPA), 2 µg/ml of the anti-ErbB2 antibody Herceptin™ (A), 2 µg/ml of the anti-EGF receptor antibody c225 (B), or a combination of valproic acid and antibodies at the same concentrations as indicated. The relative number of viable cells was determined using the enzymatic MTT assay as described in Example 6. Each point represents the mean of a set of data determined in triplicate.

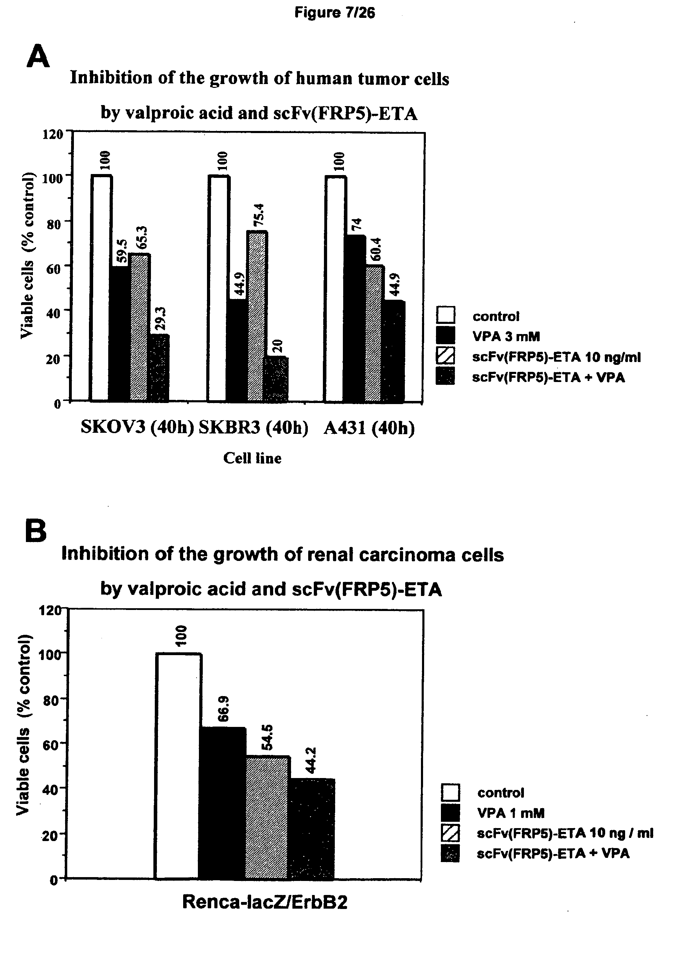

**Figure 7** shows the at least additive reduction in cellular viability of valproic acid and the immunotherapeutic/cytotoxic approach using an recombinant anti-ErbB2 immunotoxin (Example 7).
SKOV3 ovarian carcinoma cells, SKBR3 breast carcinoma cells and A431 squamous cell carcinoma cells (A) or Renca-lacZ/ErbB2 renal carcinoma cells (B) were incubated with 3mM (A) or 1mM (B) valproic acid (VPA), 10 ng/ml of recombinant anti-ErbB2 immunotoxin scFv(FRP5)-ETA, or a combination of valproic acid and scFv(FRP5)-ETA at the same concentrations as indicated. The relative number of viable cells was determined using the enzymatic MTT assay as described in Example 6. Each point represents the mean of a set of data determined in triplicate.

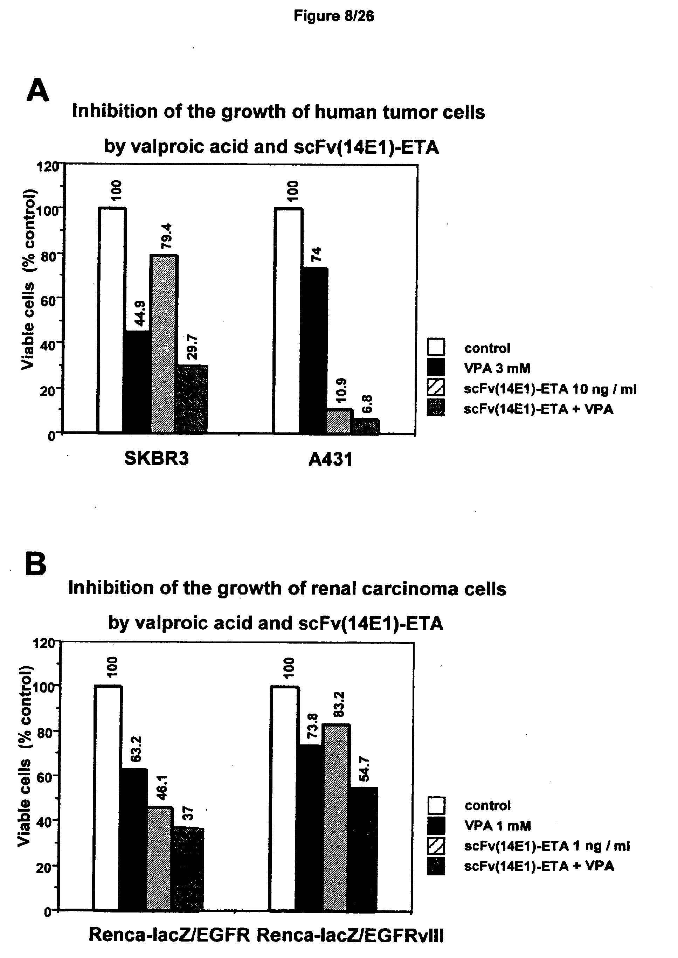

**Figure 8** shows the at least additive reduction in cellular viability of valproic acid and an immunotherapeutic/cytotoxic approach using a recombinant anti-EGF receptor immunotoxin (Example 7).
SKBR3 breast carcinoma cells and A431 squamous cell carcinoma cells (A) or Renca-lacZ/EGFR and Renca-lacZ/EGFRvIII renal carcinoma cells (B) were incubated with 3mM (A) or 1mM (B) valproic acid (VPA), 10 ng/ml (A) or 1 ng/ml (B) of recombinant anti-EGF receptor immunotoxin scFv(14E1)-ETA, or a combination of valproic acid and scFv(14E1)-

ETA at the same concentrations as indicated. The relative number of viable cells was determined using the enzymatic MTT assay as described in Example 6. Each point represents the mean of a set of data determined in triplicate.

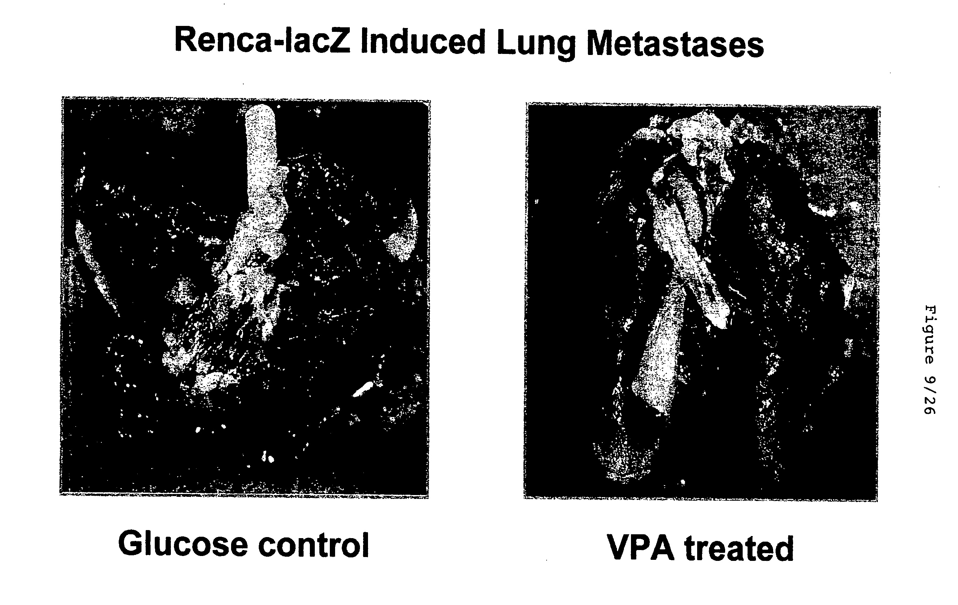

**Figure 9** shows the effect of VPA in an in vivo mouse model starting with circulating renal carcinoma cells, as an inhibitor of the development of tumor metastasis and thus of minimal residual tumor disease (Example 8).

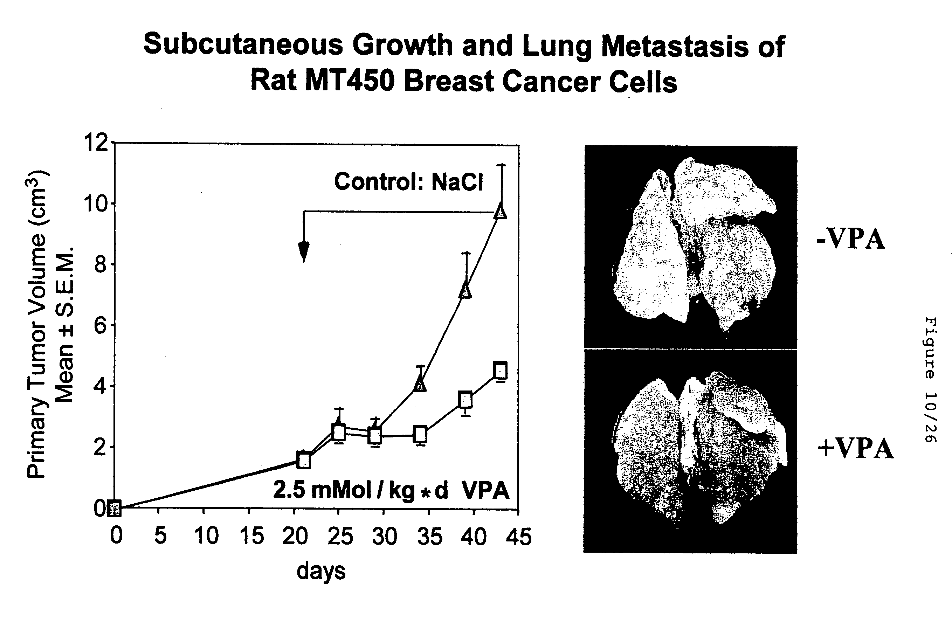

**Figure 10** shows the inhibition of subcutaneous tumor development and lung metastasis development of MT450 breast cancer cells and thus of minimal residual disease in the rat by VPA (Example 9).

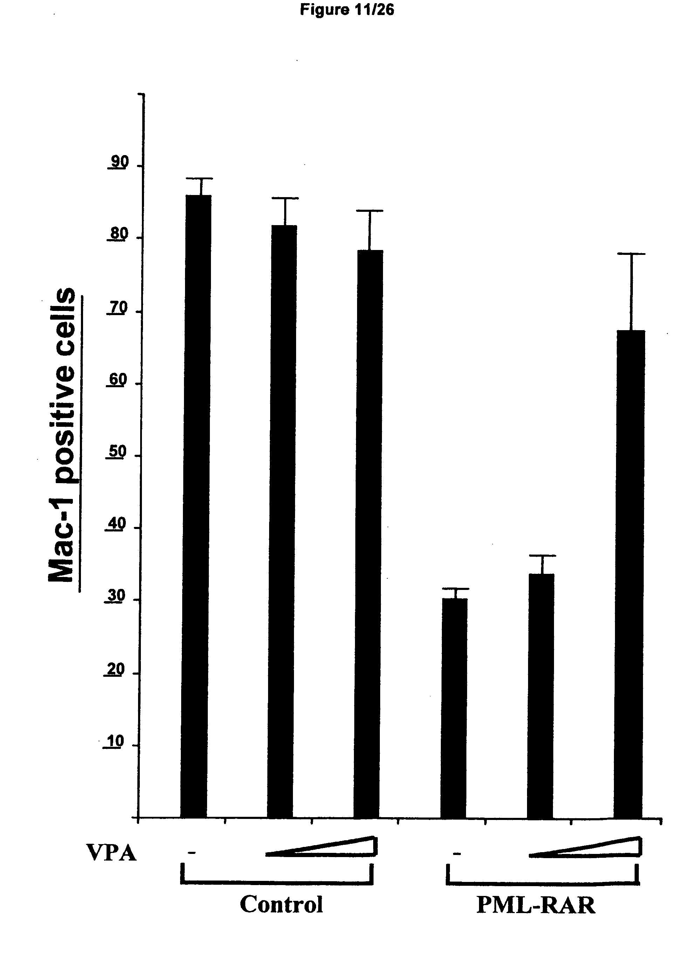

**Figure 11** shows the effect of VPA on the differentiation block of hematopoietic progenitors in vitro: VPA cooperates with cytokines in restoring the differentiative potential of PML-RAR expressing cells (Example 10) and must therefore be regarded as a sensitizing and synergistically acting reagent for the differentiation inducing activity of cytokines.

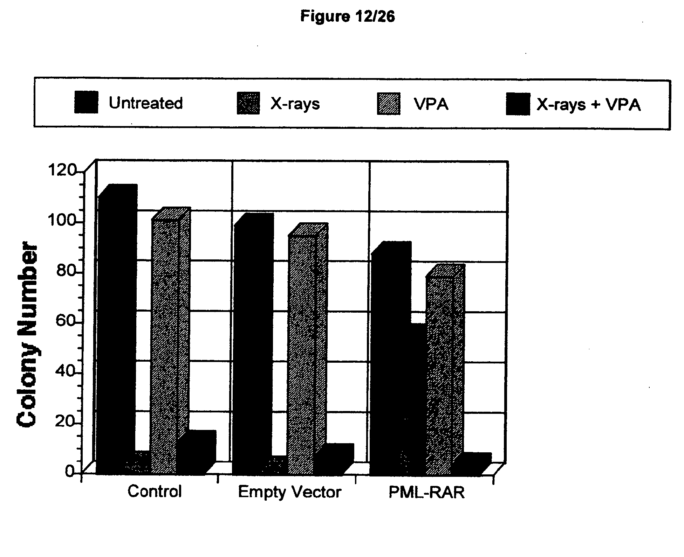

**Figure 12** shows that VPA sensitizes PML-RAR expressing cells to X-ray treatment in vitro (Example 11) and causes synergistic therapeutic activities in this combination.

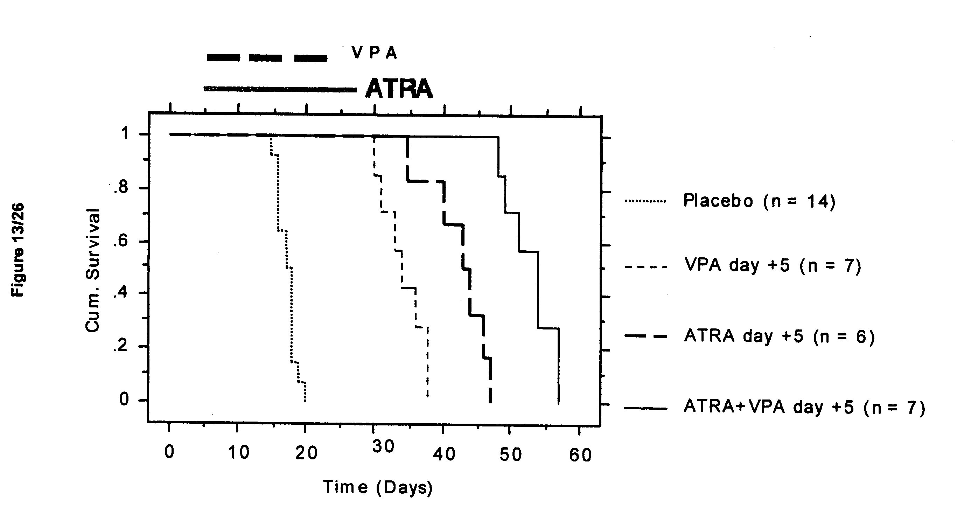

**Figure 13** shows that VPA cooperates synergistically with retinoic acid in extending survival of mice suffering from acute promyelocytic leukemia (Example 12).

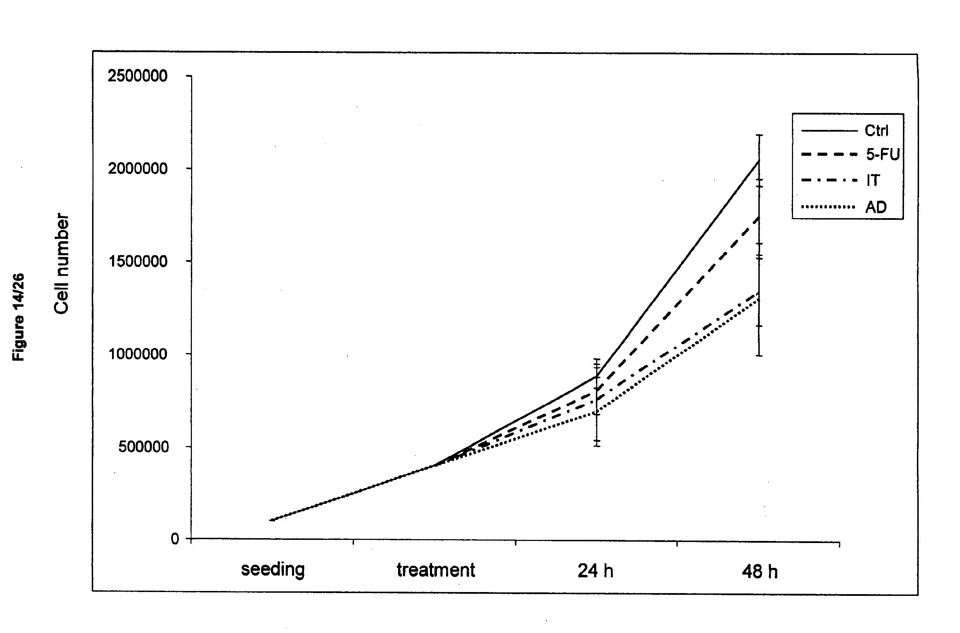

**Figure 14** shows the effect of several chemotherapeutic drugs, i.e. 5-FU, adriamycin and irinothecan alone on the cell number of HCT-116 colon cancer cells (Example 13).

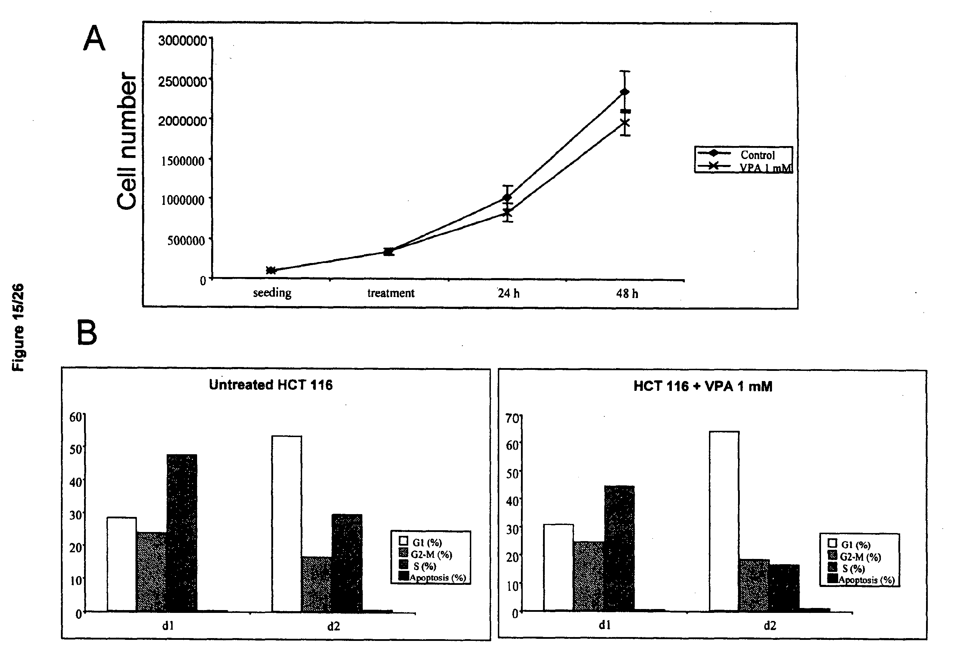

**Figure 15** shows the effect of VPA alone on the cell cycle distribution of HCT-116 colon cancer cells (Example 13).

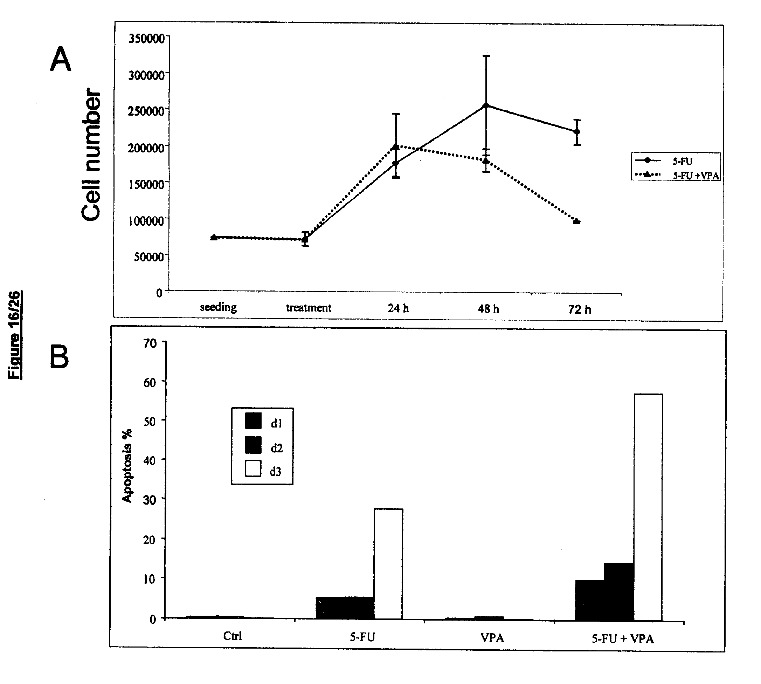

**Figure 16** shows the induction of apoptosis in HCT-116 colon cancer cells upon treatment with VPA alone and its synergistic activity in combination with the chemotherapeutic drug 5-FU (Example 13).

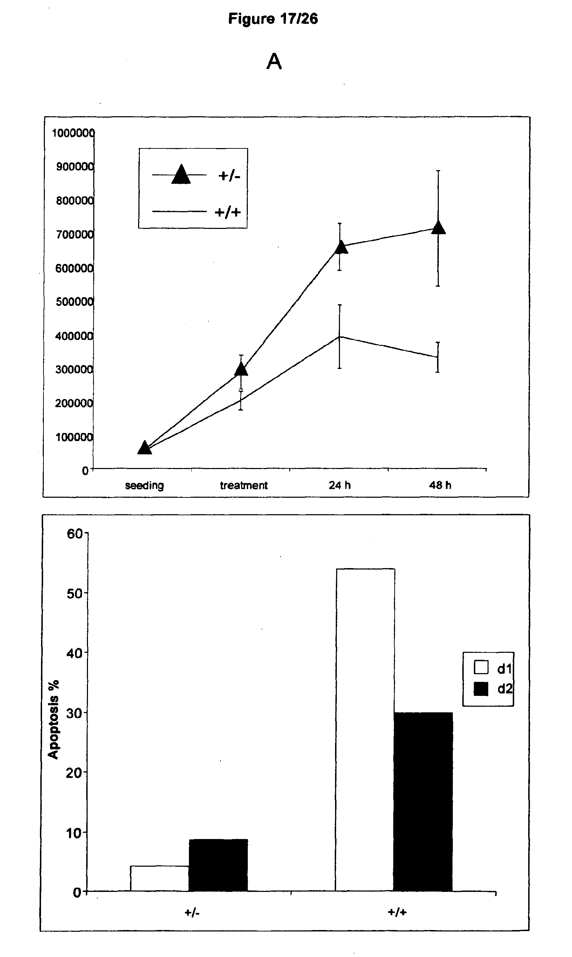

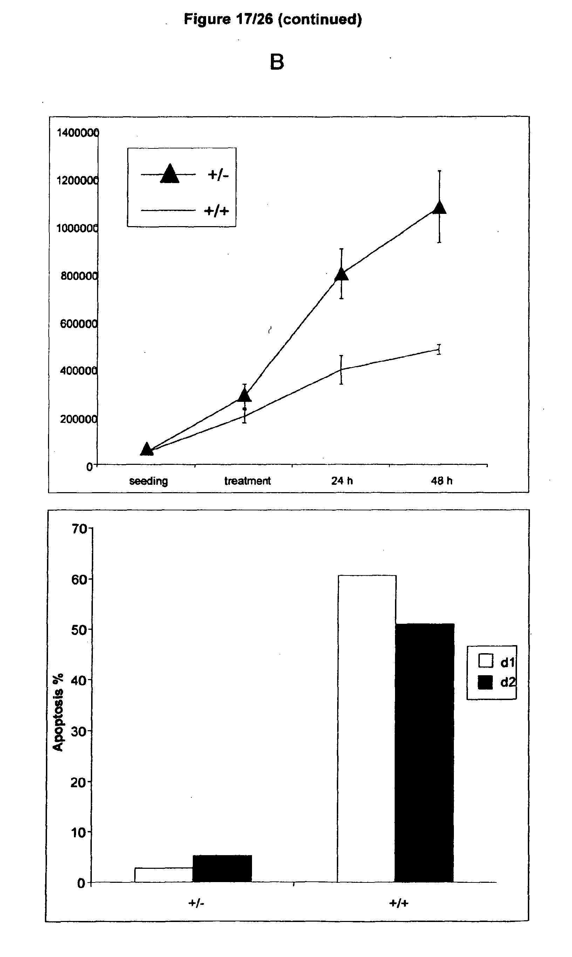

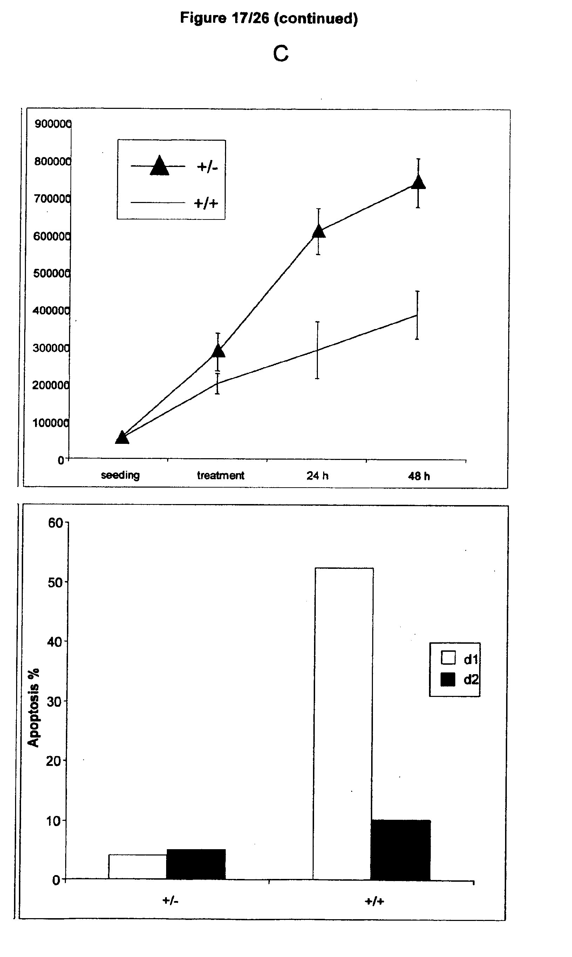

**Figure 17** shows the synergistic effect of VPA in combination with several chemotherapeutic drugs, i.e. 5-FU, adriamycin and irinothecan on the cell viability of HCT-116 colon cancer cells (Example 13).

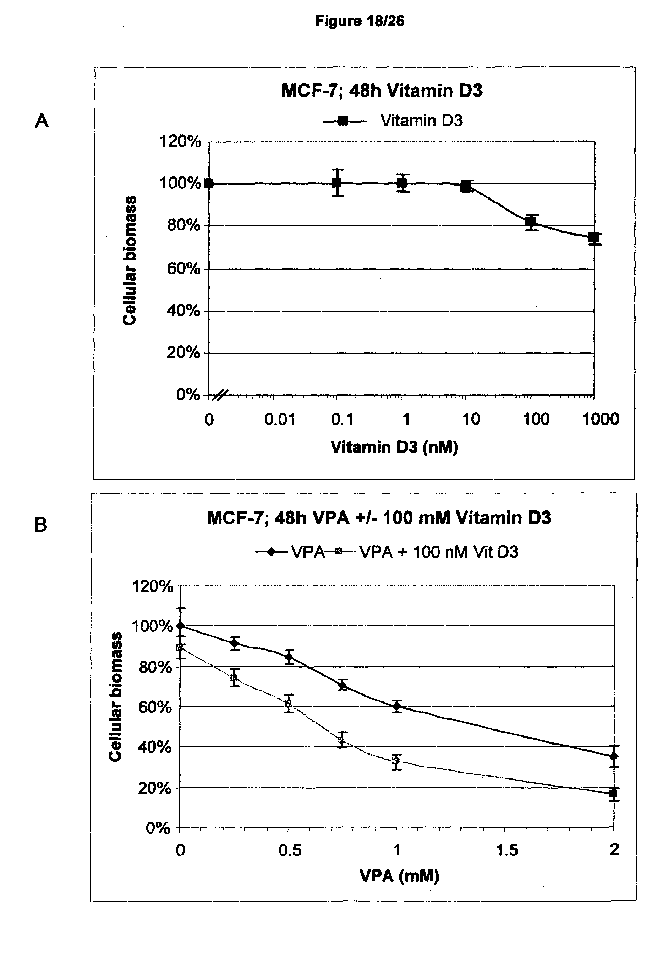

**Figure 18** shows the additive and/or synergistic reduction of cellular biomass of MCF-7 breast cancer cells by VPA in combination with the differentiation inducing drug 1α,25 Dihydroxyvitamin D₃ (Example 14).

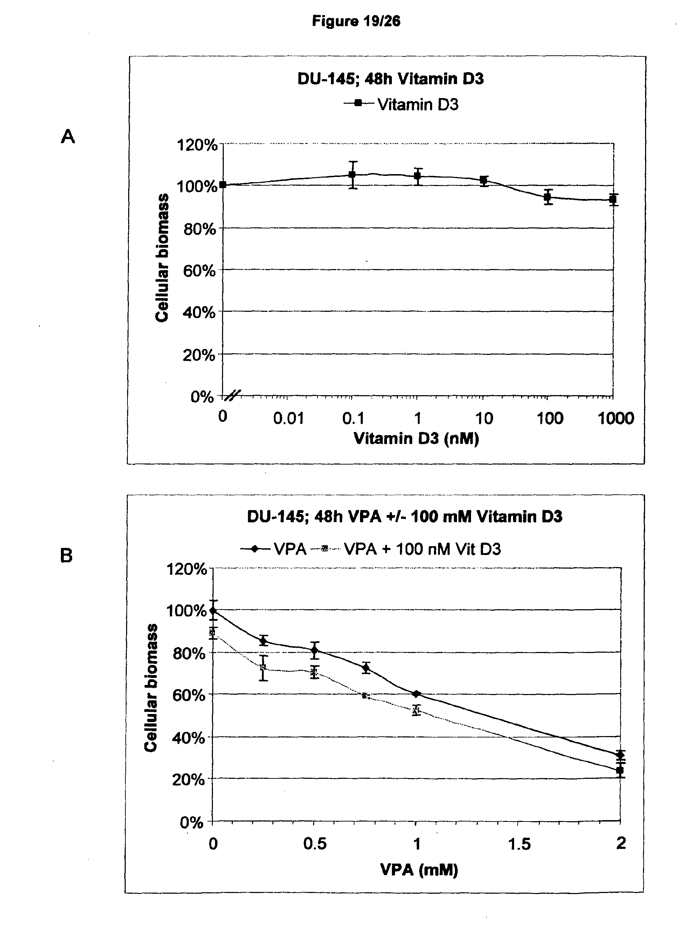

**Figure 19** shows the at least additive reduction of cellular biomass of DU-145 prostate cancer cells by VPA in combination with the differentiation inducing drug 1α,25 Dihydroxyvitamin D₃ (Example 14).

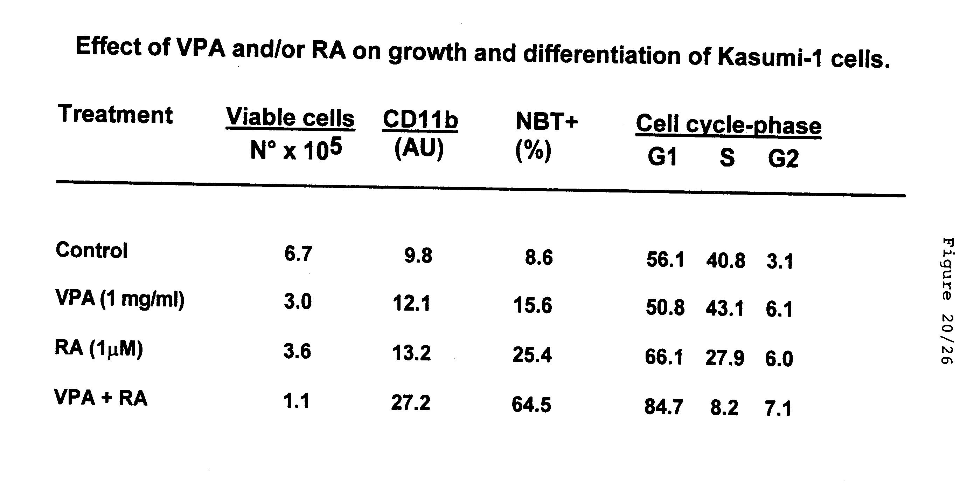

**Figure 20** shows the additive effect of VPA in combination with RA on viability and its synergistic effect on differentiation processes of Kasumi 1 cells (Example 15).

**Figure 21** shows the dose-dependent effect of VPA as single agent or in combination with RA on viability, cell number and myeloid differentiation of Kasumi 1 cells. Cell numbers were evaluated and quantified by direct cell counting (trypan blue dye exclusion method) using a hematocytometer chamber and light microscopy. Morphological examination was performed by Wright-Giemsa stained cytospins; nitroblue tetrazolium (NBT) dye reduction assay, respectively. Each point represents the mean of a set of data determined in triplicate (Example 15).

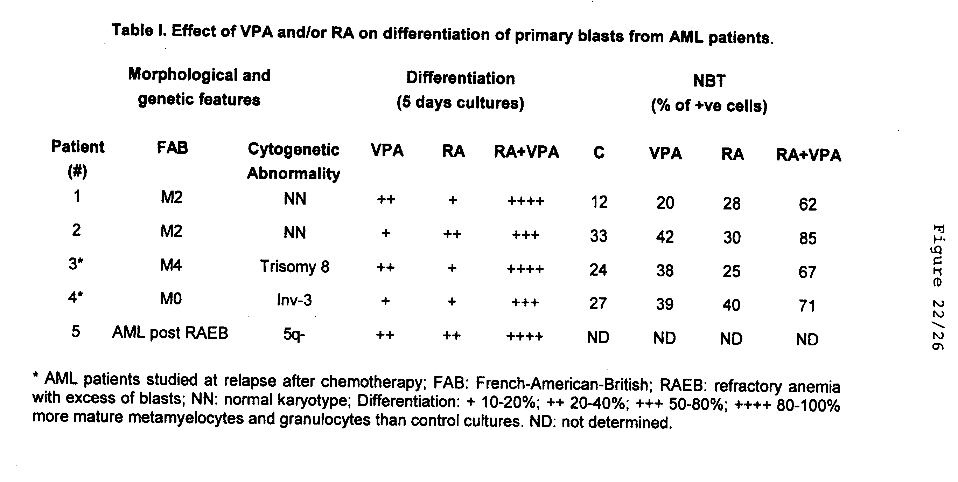

**Figure 22** shows the synergistic efficacy of ex vivo differentiation of leukemic blasts cells from AML patients upon treatment with a combination of VPA and RA compared to the use of these drugs alone (Example 15).

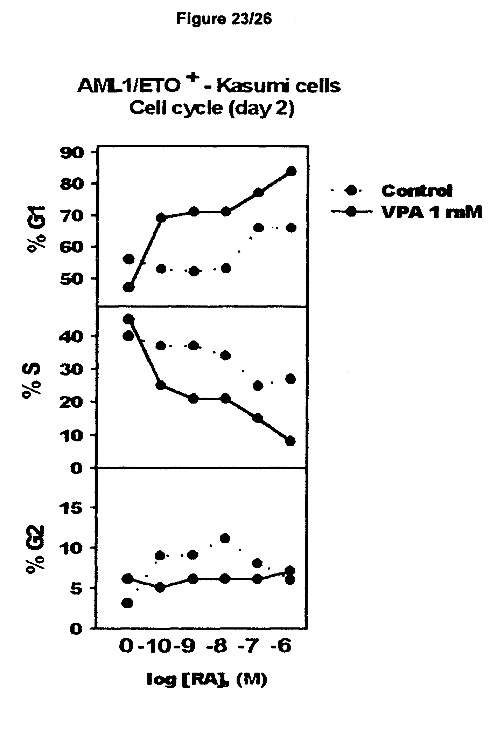

**Figure 23** shows the dose-dependent effect of RA as single agent or in combination with VPA on the cell cycle analyzed by FACS analysis of propidium-iodide stained Kasumi 1 cells (Example 15).

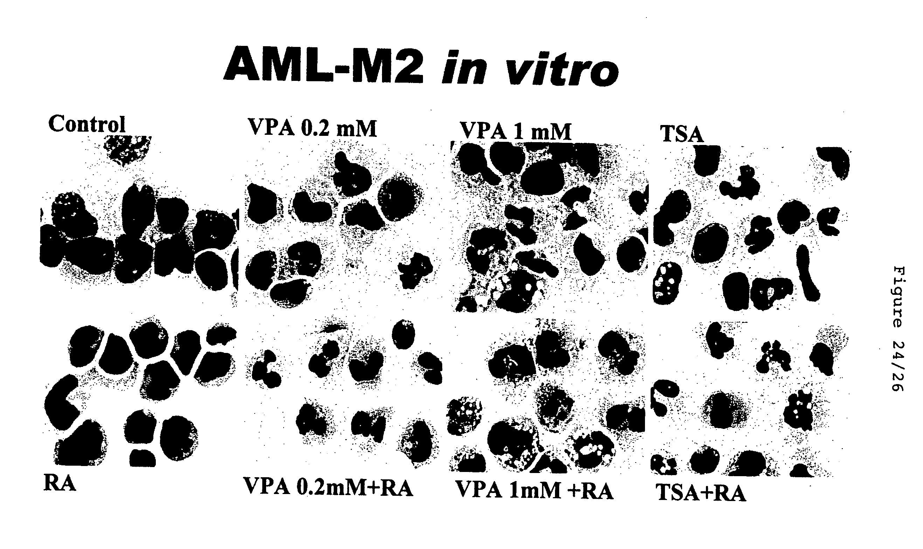

**Figure 24** shows VPA treatment alone or in combination with RA induced morphological differentiation of AML blasts (appearance of cells with metamyelocyte- or neutrophil-like morphology) (Example 15).

**Figure 25** shows the effect of histone deacetylase inhibitors TSA and VPA +/- RA on the viability of primary AML blasts as evaluated by the trypan blue dye exclusion method using a hematocytometer chamber. Each point represents the mean of a set of data determined in triplicate (Example 15). VPA may cause synergistic therapeutic responses.

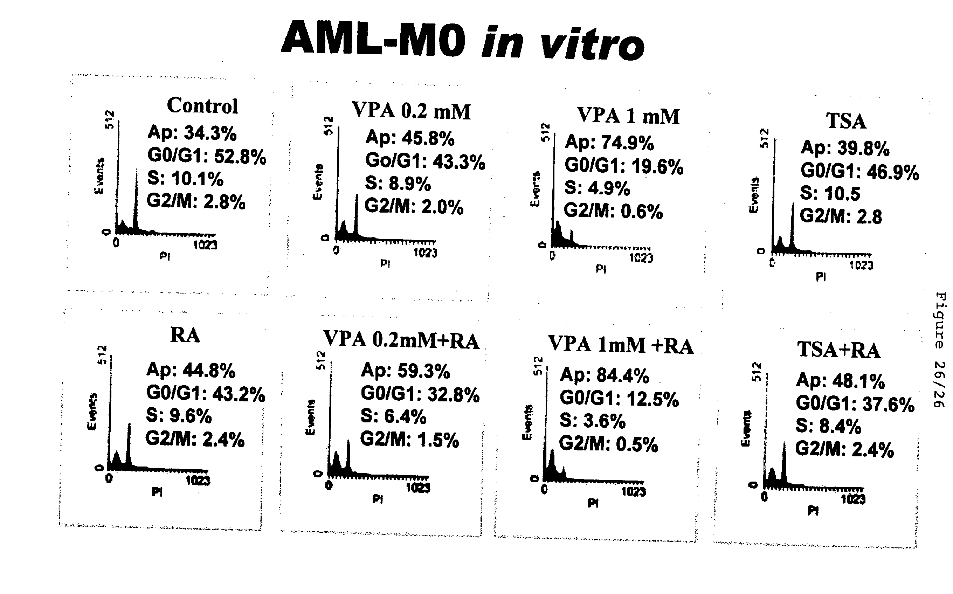

**Figure 26** shows the analysis of cell cycle changes and apoptotic DNA after treatments with VPA and TSA alone, or in combination with RA in primary AML blasts by FACS analysis of propidium-iodide stained cells (Example 15).

The following examples further illustrate the invention:

### Example 1

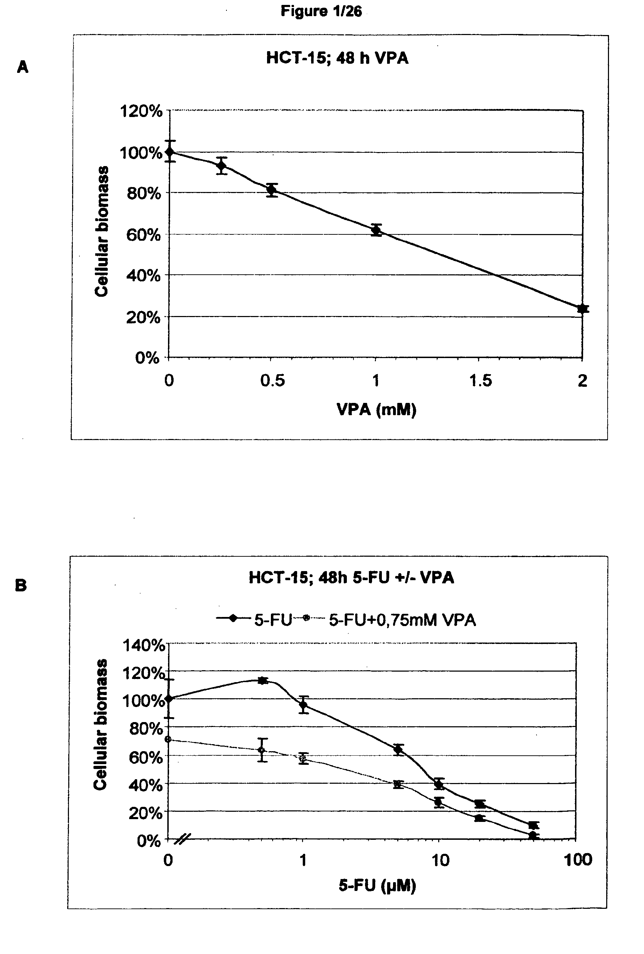

Synergistic reduction in total cellular biomass of HCT-15 colon cancer cells upon treatment with VPA or the chemotherapeutic/cytotoxic drug 5-Fluorouracil alone and by the combination of VPA and 5-Fluorouracil (5-FU) (Figure 1).

### Method:

The reduction in cellular biomass was measured by SRB-assay. For this assay cells were seeded in 96 well culture dishes at densities between 3000 and 8000 cells per well. After recovery of 24 hours they were cultured for 48 hours in the absence or presence of the indicated concentrations of VPA. Cultures were fixed with cold TCA producing a final TCA concentration of 10%. After 1 hour of incubation at 4°C the cells were washed five times with water and air dried. Fixed cells were stained for 30 minutes with 0,4% (wt/vol) Sulforhodamine B (SRB) dissolved in 1% acetic acid and washed four times with 1% acetic acid to remove unbound dye. After air drying bound dye was solubilized with 10 mM unbuffered Tris base (pH 10,5) for 5 minutes. Optical densities were read on a Titertek Multiskan Plus spectrophotometric plate reader at 550 nm. Six test wells for each dose-response were set in parallel with 12 growth control wells per cell line. A measure of the cell population density at time 0 (T₀; the time at which the drug was added) was also made from 12 extra reference wells of cells fixed with TCA just prior to drug addition to the test plates. Background OD of complete medium with 5% FBS fixed and stained as described above was also determined in 12 separate wells. From the unprocessed OD data from each microtiter plate the background OD measurements (i.e. OD of complete medium plus stain and OD of cells at T₀) were subtracted thus giving the reduction of cellular biomass of the cells.

### Results:

HCT-15 cells were cultured for 48 hours in the absence or presence of the indicated concentrations of VPA alone (Figure 1A) or in the absence or presence of the indicated concentrations of 5-FU alone or in combination with 0.75mM VPA (Figure 1B). A synergistic reduction in cellular biomass was observed upon combinatorial treatment with VPA and 5-FU together compared to treatment with VPA or 5-FU alone. This was in particular obvious when lower concentrations of 5-FU were used. E.g. 5-FU used alone at doses lower than 0.5 µM caused even an increase of the cellular biomass observed whereas the same doses of 5-FU in combination with 0.75mM VPA resulted in a stronger decrease in cellular biomass than the use of 0.75mM VPA alone. Thus, this combinatorial activity of these two drugs must be explained via a synergistic activity of this treatment.

### Example 2

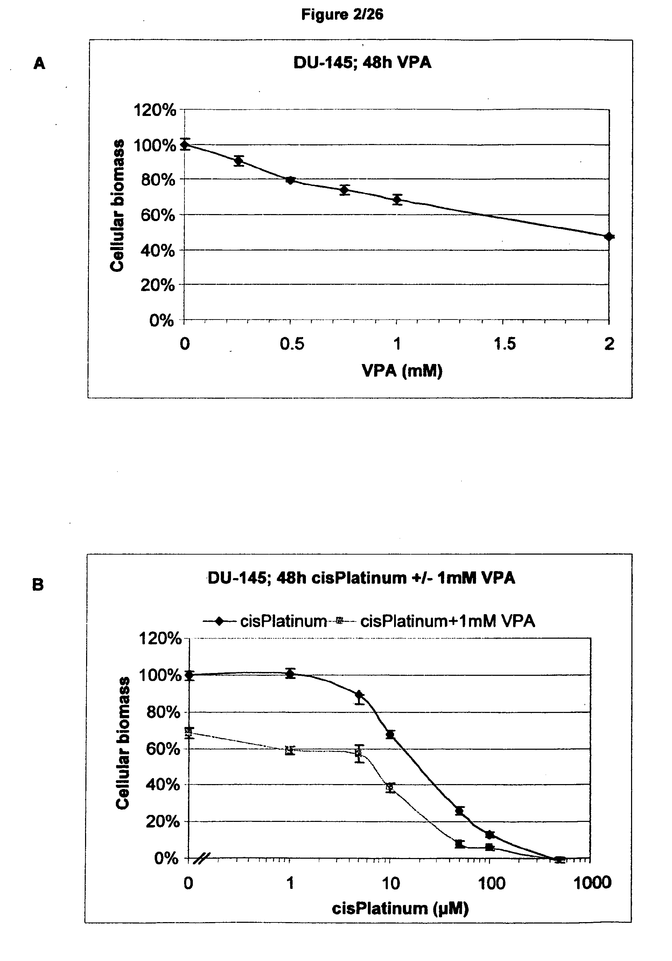

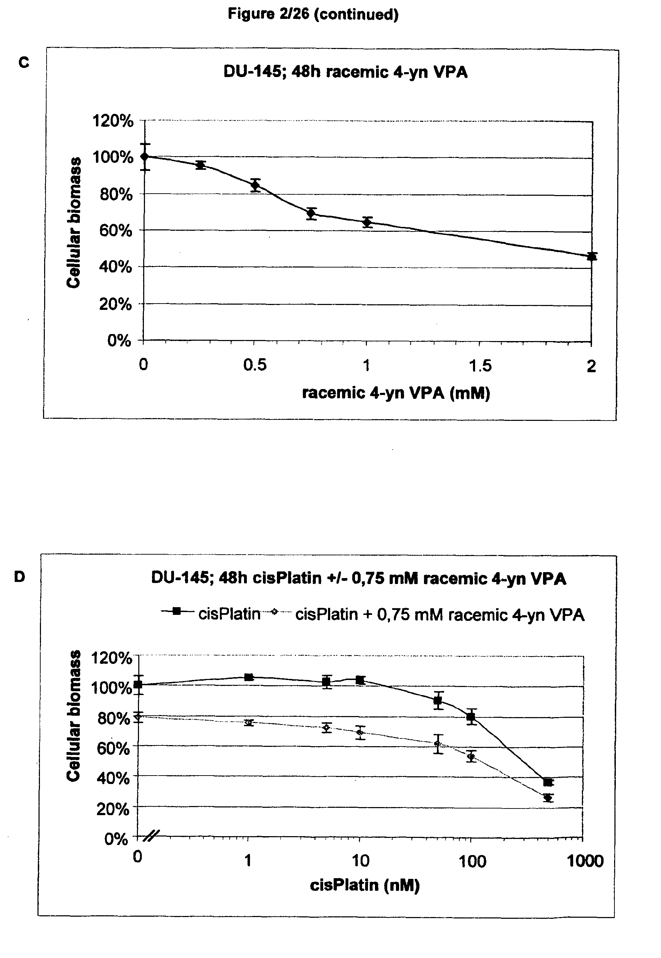

Synergistic reduction in total cellular biomass of DU-145 prostate cancer cells upon treatment with VPA or the chemotherapeutic/cytotoxic drug cisPlatinum alone and by the combination of VPA and cisPlatinum (Figure 2A-B) and the synergistic reduction in total cellular biomass of DU-145 cells achieved by the treatment with racemic 2-n-Propyl-4-pentynoic acid (4-yn VPA), a derivative of VPA, in combination with the chemotherapeutic/cytotoxic drug cisPlatinum (Figure 2C-D).

The effect on the reduction in total cellular biomass was measured by SRB-assay (see example 1 for assay and read-out procedure details). DU-145 cells were cultured for 48 hours in the absence or presence of the indicated concentrations of VPA alone (Figure 2A) or in the absence or presence of the indicated concentrations of cisPlatinum alone or in combination with 1mM VPA (Figure 2B).
Particularly when lower concentrations of cisPlatinum were used there was a synergistic reduction of cellular biomass observed, since cisPlatinum used alone at doses lower than 1 µM caused no decrease of the cellular biomass observed. In contrast, the same doses of cisPlatinum in combination with 1mM VPA resulted in a decrease in cellular biomass compared to the use of 1 mM VPA alone (Figure 2B). Thus, this combinatorial activity of these two drugs must be explained via a synergistic activity of this treatment.

In addition and in the same way DU-145 cells were cultured for 48 hours in the absence or presence of the indicated concentrations of VPA alone (Figure 2C) or in the absence or presence of the indicated concentrations of cisPlatinum alone or in combination with the VPA derivative racemic 2-n-Propyl-4-pentynoic acid (4-yn VPA) (Figure 2D). Here, particularly when concentrations lower than 10 µM of cisPlatinum were used there was a synergistic reduction of cellular biomass observed, since cisPlatinum used alone at these doses caused no decrease of the cellular biomass. In contrast, the same doses of cisPlatinum in combination with 0.75mM racemic 4-yn VPA resulted in a decrease in cellular biomass compared to the use of 0.75mM 4-yn VPA alone (Figure 2D). Thus, this combinatorial activity of these two drugs must be explained via a synergistic activity of this treatment.

### Example 3

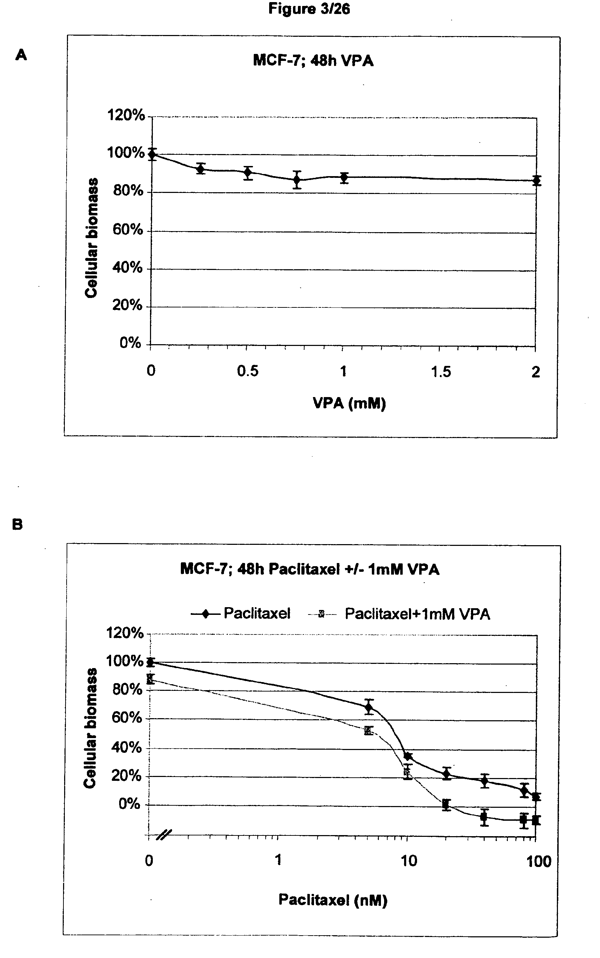

Reduction in total cellular biomass of MCF-7 estrogen-dependent breast cancer cells upon treatment with VPA or the chemotherapeutic/cytotoxic drug Paclitaxel alone and by the combination of VPA and Paclitaxel (Figure 3).

The effect on the reduction in total cellular biomass was measured by SRB-assay (see example 1 for assay and read-out procedure details). MCF-7 cells were cultured for 48 hours in the absence or presence of the indicated concentrations of VPA alone (Figure 3A) or in the absence or presence of the indicated concentrations of Paclitaxel alone or in combination with 0.75mM VPA (Figure 3B).
A clear additive reduction in cellular biomass was observed upon combinatorial treatment with VPA and Paclitaxel at the same time compared to treatment with VPA or Paclitaxel alone.

### Example 4

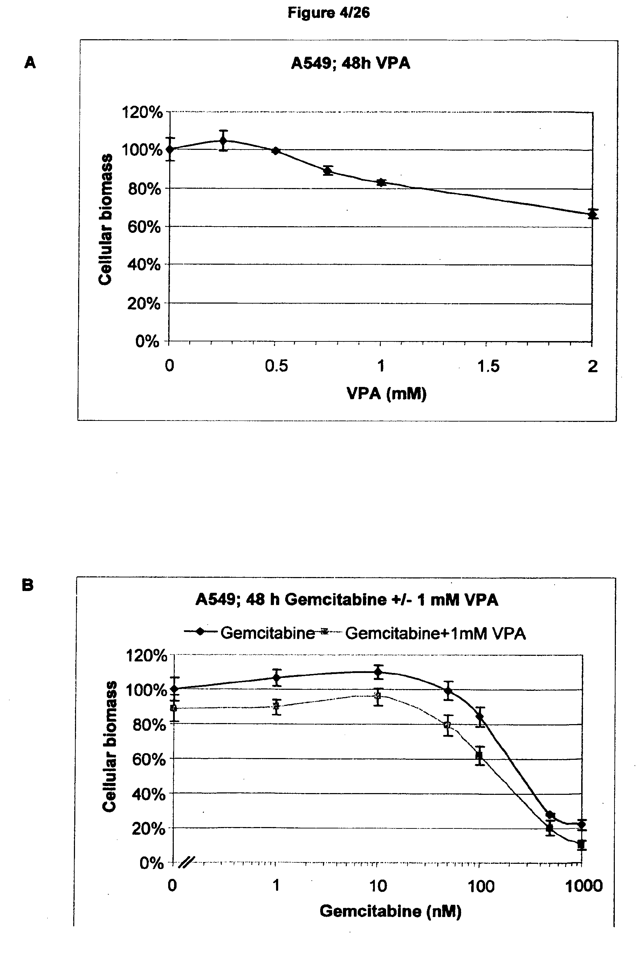

Reduction in total cellular biomass of A-549 non-small cell lung cancer cells upon treatment with VPA or the chemotherapeutic/cytotoxic drug Gemcitabine alone and by the combination of VPA and Gemcitabine (Figure 4).

The effect on the reduction in total cellular biomass was measured by SRB-assay (see example 1 for assay and read-out procedure details). A-549 cells were cultured for 48 hours in the absence or presence of the indicated concentrations of VPA alone (Figure 4A) or in the absence or presence of the indicated concentrations of Gemcitabine alone or in combination with 0.75mM VPA (Figure 4B).
A clear additive reduction in cellular biomass was observed upon combinatorial treatment with VPA and Gemcitabine at the same time compared to treatment with VPA or Gemcitabine alone.

### Example 5

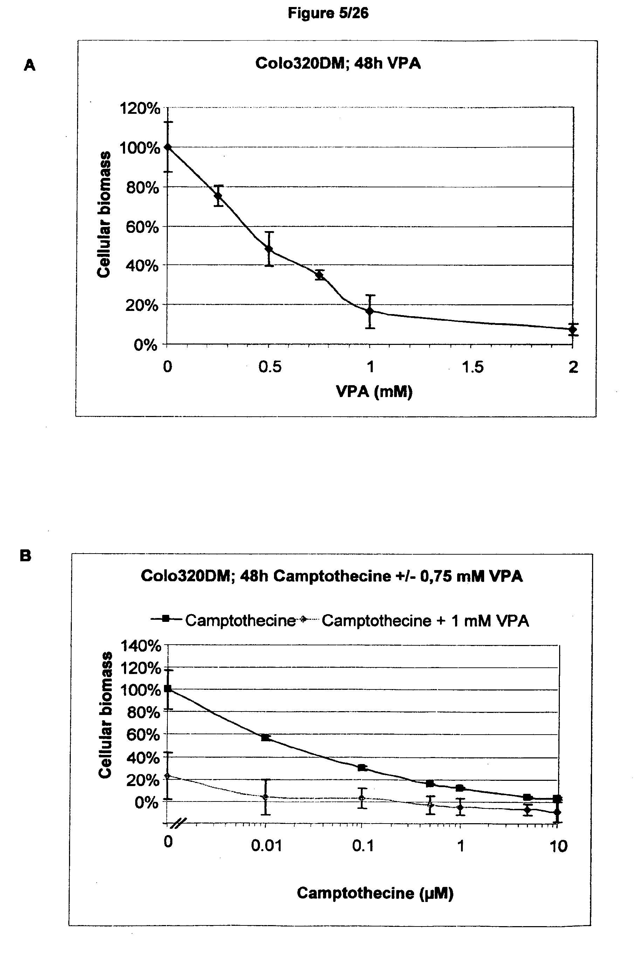

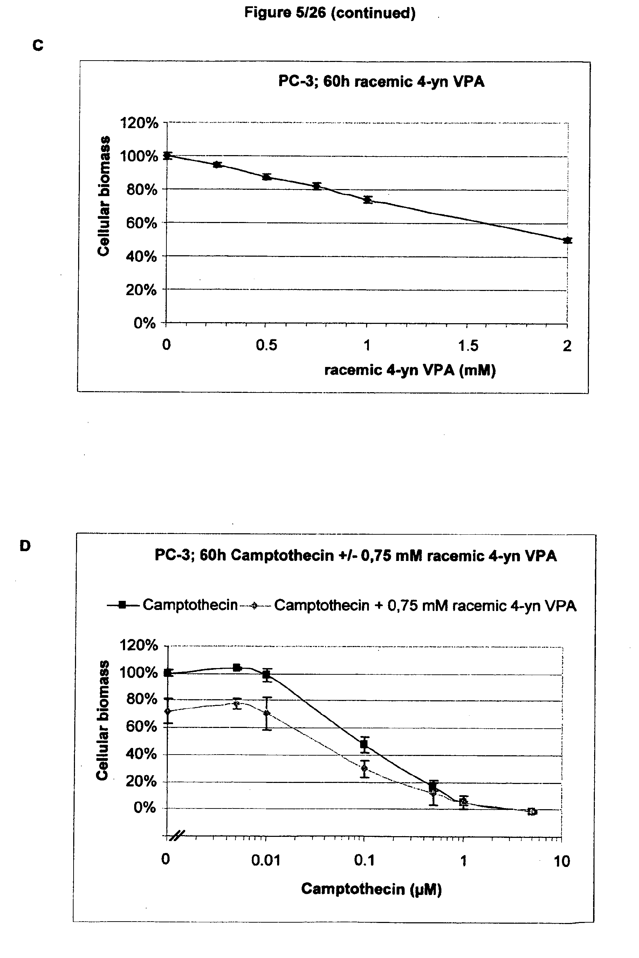

The reduction in total cellular biomass of COLO320DM colon cancer cells upon treatment with VPA or the chemotherapeutic/cytotoxic drug Camptothecin alone and by the combination of VPA and Camptothecin (Figure 5A-B) is shown and the at least additive reduction in total cellular biomass of PC-3 prostate cancer cells achieved by the treatment with racemic 2-n-Propyl-4-pentynoic acid (4-yn VPA), a derivative of VPA (Figure 5C-D), in combination with the chemotherapeutic drug Camptothecin is presented.

The effect on the reduction in total cellular biomass was measured by SRB-assay (see example 1 for assay and read-out procedure details). COLO329DM or PC-3 cells were cultured for 48 hours in the absence or presence of the indicated concentrations of VPA (Figure 5A) or racemic 4-yn VPA alone (Figure 5C) or in the absence or presence of the indicated concentrations of Camptothecin alone or in combination with 0.75mM VPA (Figure 5B) or with 0.75mM of racemic 4-yn VPA (Figure 5D).
A clear reduction in cellular biomass was observed in both cases of combinatorial treatment, indicating that not only VPA in combination with Camptothecin, but also its derivative 4-yn VPA has the same additional suitable effect leading to an at least additive therapeutic effect when it is combined with other anti-cancer drugs, as exemplified here for Campthothecin.

### Example 6

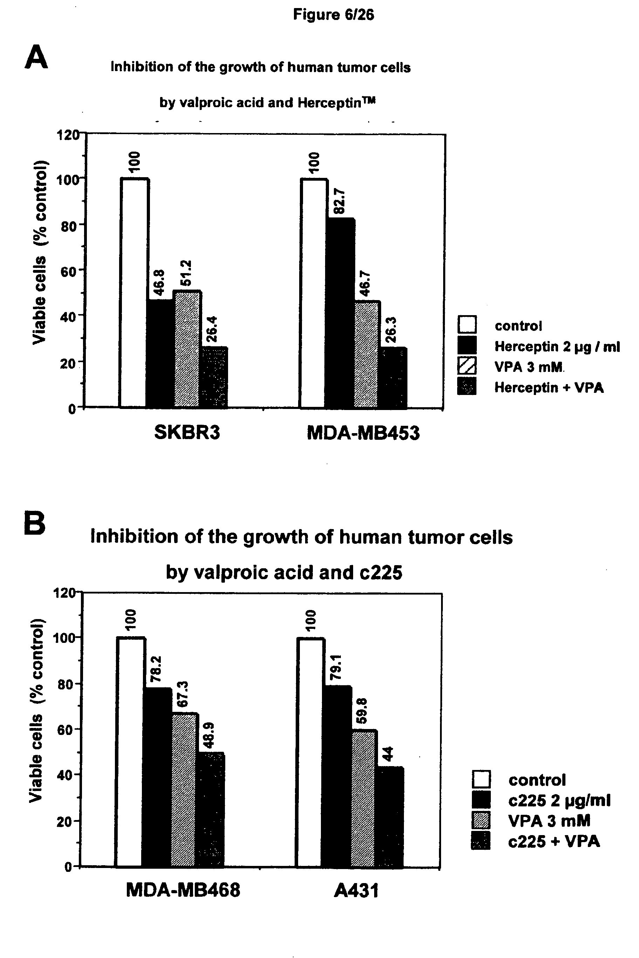

Synergistic and/or additive inhibition of tumor cell viability by valproic acid in combination with monoclonal antibodies as immunotherapeutic agents (Figure 6).

### Cell lines and cell culture:

Human MDA-MB468, MDA-MB453 and SKBR3 breast carcinoma cells and A431 squamous cell carcinoma cells were maintained in Dulbecco's modified Eagle's medium (DMEM, BioWhittaker, Verviers, Belgium) supplemented with 10% heat inactivated fetal bovine serum (FBS), 2 mM L-glutamine, 100 units/ml penicillin, and 100 µg/ml streptomycin.

### Cell viability assays:

Tumor cells were seeded in 96 well plates at a density of 1 x 10⁴ cells/well in normal growth medium. Cells were treated for 70 h with valproic acid at a final concentration of 3 mM, or 2 µg/ml of the therapeutic anti-ErbB2 antibody Herceptin™, or 2 µg/ml of the therapeutic anti-EGF receptor antibody c225 (Fan & Mendelsohn, Curr. Opin. Oncol., 10: 67-73, 1998), or a combination of valproic acid with either Herceptin™ or c225 antibodies at the same concentrations. Control cells were grown in the absence of valproic acid or antibodies. 10 µl of 10 mg/ml 3-(4,5-dimethylthiazole-2-yl)-2,5 diphenyltetrazolium bromide (MTT) (Sigma, Deisenhofen, Germany) in PBS were added to each well and the cells were incubated for another 3 h. Cells were lysed by the addition of 90 µl of lysis buffer (20% SDS in 50% dimethyl formamide, pH 4.7). After solubilization of the formazan product, the absorption at 590 nm was determined in a microplate reader (Dynatech, Denkendorf, Germany) and the relative amount of viable cells in comparison to cells cultured without the addition of valproic acid or antibodies was calculated.

### Results:

The results presented in Figure 6 show that valproic acid and therapeutic antibodies Herceptin™ and c225 as a single reagent each inhibit the viability of breast carcinoma cells and squamous cell carcinoma cells. However, combination treatment with valproic acid and the therapeutic antibody Herceptin™ in SKBR3 cells resulted in a pronounced additive therapeutic effect. But more intriguingly a synergistic reduction of cell viability caused by combinatorial treatment with VPA was observed in three other cell lines tested, namely in combination with Herceptin™ in MDA-MB453 cells, and in combination with c225 in MDA-MB468 and in A431 cells. These results demonstrate that valproic acid in combination with therapeutic antibodies displays a strongly enhanced therapeutic effect and potently inhibits the viability of a variety of tumor cells derived from solid tumors of epithelial origin. Furthermore, the results indicate that valproic acid and derivatives could be used in combination with therapeutic antibodies for the therapy of such tumors with synergistic therapeutic success.

### Example 7

Inhibition of tumor cell growth by valproic acid and an immunotherapeutic/cytotoxic approach using recombinant antibody fusion proteins (Figures 7 and 8).

### Cell lines and cell culture:

Human SKBR3 breast carcinoma cells, A431 squamous cell carcinoma cells and SKOV3 ovarian carcinoma cells were maintained in Dulbecco's modified Eagle's medium (DMEM, BioWhittaker, Verviers, Belgium) supplemented with 10% heat-inactivated fetal bovine serum (FBS), 2 mM L-glutamine, 100 units/ml penicillin, and 100 µg/ml streptomycin.
Renal cell carcinoma (Renca) cells stably transfected with plasmid pZeoSV2/lacZ encoding E. coli β-galactosidase, and either with plasmids pSV2ErbB2N and pSV2neo encoding c-erbB2 and neomycin resistance (Renca-lacZ/ErbB2) (Maurer-Gebhard et al., Cancer Res. 58: 2661-2666, 1998), or plasmids pLTR-EGFR or pLTR-EGFRvIII and pSV2neo encoding epidermal growth factor (EGF) receptor, the oncogenically activated EGF receptor variant EGFRvIII, and neomycin resistance (Renca-lacZ/EGFR and Renca-lacZ/EGFRvIII) (Schmidt et al., Oncogene 18: 1711-1721, 1999) were grown in RPMI-1640 medium supplemented with 8% FBS, 2 mM L-glutamine, 100 U/ml penicillin, 100 µg/ml streptomycin, 0.25 mg/ml Zeocin and 0.48 mg/ml G418.

### Cell viability assays:

Tumor cells were seeded in 96 well plates at a density of 1 x 10⁴ cells/well in normal growth medium. SKBR3, A431 and SKOV3 cells were treated for 40 h with valproic acid at a final concentration of 3 mM, or 10 ng/ml of the recombinant anti-ErbB2 single chain antibody-toxin scFv(FRP5)-ETA (Wels et al., Cancer Res. 52: 6310-6317, 1992), or 10 ng/ml of the recombinant anti-EGF receptor single chain antibody-toxin scFv(14E1)-ETA (Schmidt et al., Brit. J. Cancer 75: 1575-1584, 1997), or a combination of valproic acid with either scFv(FRP5)-ETA or scFv(14E1)-ETA at the same concentrations. Renca-lacZ/ErbB2, Renca-lacZ/EGFR and Renca-lacZ/EGFRvIII cells were treated for 40 h with valproic acid at a final concentration of 1mM, or 10 ng/ml of the recombinant anti-ErbB2 single chain antibody-toxin scFv(FRP5)-ETA, or 1 ng/ml of the recombinant anti-EGF receptor single chain antibody-toxin scFv(14E1)-ETA, or a combination of valproic acid with either scFv(FRP5)-ETA or scFv(14E1)-ETA at the same concentrations. Control cells were grown in the absence of valproic acid or antibody-toxins. Ten µl of 10 mg/ml 3-(4,5-dimethylthiazole-2-yl)-2,5 diphenyltetrazolium bromide (MTT) (Sigma, Deisenhofen, Germany) in PBS were added to each well and the cells were incubated for another 3 h. Cells were lysed by the addition of 90 µl of lysis buffer (20% SDS in 50% dimethyl formamide, pH 4.7). After solubilization of the formazan product, the absorption at 590 nm was determined in a microplate reader (Dynatech, Denkendorf, Germany) and the relative amount of viable cells in comparison to cells cultured without the addition of valproic acid or antibody-toxins was calculated.

Results:
The results presented (Figure 7 and 8) show that valproic acid and anti-ErbB2 or anti-EGF receptor immunotoxins as a single reagent each inhibit the viability of breast, ovarian, renal and squamous cell carcinoma cells. However, combination treatment of the cells with valproic acid and anti-ErbB2 or anti-EGF receptor immunotoxins results in a pronounced additive therapeutic effect. These results demonstrate that valproic acid in combination with antibody fusion proteins such as immunotoxins displays a strongly enhanced therapeutic effect and potently inhibits the viability of a variety of tumor cells derived from solid tumors of epithelial origin. Furthermore, the results indicate that valproic acid and derivatives could be used in combination with immunotoxins for the therapy of such tumors.

### Example 8

VPA acts as an inhibitor of the progression of the development of tumor metastasis, thus of minimal residual disease in an in vivo mouse model of renal carcinoma (Figure 9) cells.

Mouse renal carcinoma cells (Renca) were established from a spontaneously arising kidney tumor in Balb/c mice. These cells efficiently form tumors in the lung upon transplantation into Balb/c mice through the tail vein and the tumorigenic properties of Renca cells have been well established (Murphy, et al., 1973, J. Natl. Cancer Inst., 50: 1013-1025; Hrushesky et al., 1973, J. Surg. Res., 15: 327-332; Williams et al., 1981, Res. Comm. Chem. Pathol. Pharmacol., 34: 345-349). The circulating tumor cells in this model setting represent a mimicry of situations frequently found in tumor patients when residual tumor cells are circulating in a patient's body and finally invade and home in various organs to grow as metastatic tumors. The inhibition of such minimal residual tumor diseases is one of the major aims of modern tumor therapy and can be experimentally examined in the in vivo model used here.

### Experimental setting:

Renca cells and the transfected cell clone Renca-lacZ (encoding for β-galactosidase) were grown in RPMI-1640 supplemented with 10% fetal calf serum (FCS). For metastasis formation in recipient mice 10⁵ Renca-lacZ cells in 100 µl PBS were injected into the lateral tail vein of female Balb/c mice at 4 to 6 weeks of age. Five animals per group were sacrificed in weekly intervals for up to four weeks post tumor cell injection and the lungs were excised (Maurer-Gebhard et al. 1998, Cancer Research 58, 2661-2666).

Treatment of mice and results:
Treatment by VPA was as followed: 2 x 400 mg / kg / day i.p. (Na-VPA, 155mM in H₂O)
Control animals were treated with a glucose solution (Maurer-Gebhard et al. 1998, Cancer
Research 58, 2661-2666) (Figure 9).
X-gal staining for visualization of pulmonary metastases:
Excised lungs were fixed overnight at 4°C in PBS containing 2 % formaldehyde and 0.2 % glutaraldehyde. Fixative solution was removed and lungs were washed with PBS. Staining with X-Gal solution was performed at 37°C in the dark for 10 to 12 hours (Maurer-Gebhard et al. 1998, Cancer Research 58, 2661-2666). Metastatic surface nodules were analyzed under a dissecting microscope and representative pictures were taken and are presented in figure 9.

Figure 9 shows that treatment with VPA effectively inhibited the formation of lung metastasis, i.e. the number and the size of metastatic nodules. This indicates that VPA may therapeutically be used for the inhibition of the development of metastasis arising from epithelial tumors and for the therapy of minimal residual tumor disease.

### Example 9

Inhibition of subcutaneous tumor growth and lung metastasis of MT450 breast cancer cells and thus of minimal residual disease in the rat by VPA (Figure 10).

### Experimental setting:

MT450 cells were grown in DMEM/10% FCS medium and tested for absence of mycoplasms just prior to injection. Cells were washed twice in PBS and suspended to a density of 5 x 10⁶ cells per ml of PBS. 5 x 10⁵ cells in 0.1 ml of PBS were injected into each rat. 2 groups of 8 rats each were used (for ± VPA, each). Rats were left to grow primary tumors for 21 days. VPA sodium salt was dissolved to 155 mM (isotonic) in water. pH was adjusted between 6 and 7 by a small amount of hydrochloric acid. The solution was sterile filtered. The compound was applied by i.p. injection. Each dosing was 2 ml per 250 g rat which corresponds to 1.25 mmol VPA per kg BW (body weight) and dose. Two doses per day were applied. Control animals received equal amounts of a sterile isotonic sodium chloride solution.

### Results:

The growth of the primary tumors was followed by measurement of tumor volume indicating that VPA delays tumor expansion. The experiment was terminated when tumor size in one of the rats of the control group had reached the legal limit of 50 mm. Necropsy at that time was performed on all rats of the experiment to assess lung metastasis. All rats of the control group showed significant development of metastasis. A representative example is shown in figure 10. Metastasis was also found in 7 out of 8 VPA-treated rats. However, size and number of metastases were much smaller compared to NaCl-treated rats. A representative example is shown in figure 10. A dose finding experiment had shown that the chosen dosage protocol lead to high initial serum levels (e.g. 3.6 mM at 1 hour after i.p. injection) which rapidly dropped (e.g. 0.25 mM at 4 hours after i.p. injection) below those levels which are maintained during therapy of epilepsy in human. In summary the experiment shows that even though the fast clearance of VPA from the rodent serum which does not allow to maintain VPA serum levels in the expected effective range above 0.5 mM, VPA treatment substantially decreases primary tumor growth and lung metastasis in the MT450 rat breast cancer model and thus may be used to inhibit minimal residual tumor disease.

### Example 10

Synergistic effect of VPA on the differentiation block of hematopoietic progenitors in vitro: VPA cooperates with cytokines in restoring the differentiative potential of PML-RAR expressing cells. VPA must therefore be regarded as a sensitizing reagent for the differentiation inducing activity of cytokines (Figure 11).

Since acute promyelocytic leukemia cells are known to respond to treatment with HDAC inhibitors, the effect of VPA on the differentiation block of hematopoietic precursor cells by PML-RAR was tested. Murine hematopoietic progenitors (lin-) were transduced with a retroviral vector encoding PML-RAR, and GFP as a marker. Transduced cells were stimulated to differentiate with a cocktail containing several cytokines (IL3, IL6, SCF, G-CSF and GM-CSF) in the absence or presence of VPA. Myeloid differentiation was scored by analyzing the presence of the differentiation marker Mac-1.

### Results:

VPA treatment did not affect differentiation of control cells whereas expression of PML-RAR caused a strong differentiation block (Figure 11) which could not be overcome when the cytokines described where used as the only treatment. However, VPA (1mM, in right panels, labeled PML-RAR) almost completely reverted the differentiation block imposed by PML-RAR (Figure 11). Thus, in the absence of RA, VPA re-establishes and sensitizes the cells for a state permissive for differentiation, then induced by cytokines, presumably by inhibiting the action of the HDAC complex recruited to target genes by PML-RAR. This activity of VPA must be regarded as a synergistic activity since the treatment with cytokines alone does not lead to a significant release of the differentiation block in these PML-RAR cells as mentioned above.

### Methods:

Murine hematopoietic progenitors were purified from the bone marrow of 129 mice on the basis of the absence of lineage differentiation markers (lin-). Lin- cells were grown for 48 hours in the presence of IL-3 (20 ng/ml), IL-6 (20 ng/ml), SCF (100 ng/ml), and then attached to non-tissue culture treated plates coated with Retronectin (Takara-Shuzo). Cells were then transduced by incubation with the supernatant from Phoenix ecotropic packaging cells (supplemented with fresh serum, and IL-3, IL-6, and SCF as above), transiently transfected with the control retroviral vector PINCO, or PINCO-PML-RAR. After 60 hours, GFP+ cells were sorted by FACS, and seeded in methylcellulose plates supplemented as above, plus G-CSF (60 ng/ml) and GM-CSF (20 ng/ml). Where indicated, sodium valproate (VPA, from left to right 0.2 or 1mM) was added to the differentiation medium. After 8-10 days, cells were analyzed for the presence of the myeloid differentiation marker Mac-1 by FACS. VPA did not cause significant changes in the number of Mac-1⁺ cells, nor in the number of colonies in control cells up to concentrations of 1mM. At higher concentrations (>3 mM) a reduction in the number of colonies was observed, most likely due to induction of cell death (data not shown). As a control, the analysis of VPA-treated cells with an erythroid differentiation marker (Ter-119) did not reveal the presence of positive cells (data not shown). Uninfected cells, and cells infected with the control PINCO vector behaved identically (data not shown).

For figure 11 Lin- cells were transduced with the indicated vectors (control: PINCO, empty vector encoding GFP alone), and GFP+ cells were sorted by FACS. GFP+ cells were then plated in differentiation medium, in the absence or in the presence of VPA (from left to right 0.2 and 1mM). Differentiation was assessed after 8-10 days by analysis of the myeloid differentiation marker Mac-1.

### Example 11

VPA sensitizes PML-RAR expressing cells to X-ray treatment in vitro and causes a synergistic therapeutic effect (Figure 12).

### Results:

Upon X-ray treatment (2 Gray), hematopoietic progenitor cells show a strong decrease in their survival potential and undergo apoptosis. Semisolid culture conditions (methylcellulose-based mediums) led to an almost complete absence of colonies (derived from colony-forming cells, CFCs) in X-rays treated cultures of wild-type cells, demonstrating that undifferentiated cells are very sensitive to this treatment (Figure 12). Strikingly, PML-RAR expression caused a strong reduction in X-ray sensitivity of target cells, with a >50% rescue rate (Figure 12). Under the same conditions, VPA (1mM) slightly decreased the sensitivity of wild-type cells. However, VPA led to a re-sensitization of PML-RAR expressing cells, with a complete and clearly synergistic disappearance of colonies in VPA treated cells (Figure 12). It appears therefore, that VPA may be combined with X-rays to rescue the sensitivity of cells that have become resistant (e.g. through expression of an oncogenic fusion protein) to X-ray treatment alone and may cause synergistic therapeutic success rates.

### Methods:

Lin- cells were transduced as described for figure 11 (see also methods in example 10 for details), and sorted by FACS. 12 hours after sorting, cells were washed with PBS, and then incubated for 8-12 hours in medium with cytokines [data presented in figure 12 using IL-3 (20 ng/ml), IL-6 (20 ng/ml), SCF (100 ng/ml), G-CSF (60 ng/ml), GM-CSF (20 ng/ml)] or without cytokines (data not shown). At the end of the incubation, cells were exposed to an X-ray source (2 Gy total exposure), and incubated for additional 12-16 hours in the presence or in the absence of VPA. Finally, cells were plated in methylcellulose containing medium (StemCell Technologies) in the presence of cytokines (IL3, IL6, SCF, G- and GM-CSF) for 8-10 days.

For Figure 12 uninfected cells ("Control"), or cells expressing GFP alone ("Empty Vector"), or cells expressing GFP and PML-RAR ("PML-RAR") were used. 8 days after plating in methylcellulose, the total number of colonies was measured. VPA-treated cells (1 mM) were exposed to VPA also prior to X-ray treatment.

### Example 12

VPA cooperates in combination with retinoic acid in extending survival of mice suffering from acute promyelocytic leukemia (Figure 13).

Re-introduction of PML-RAR expressing hematopoietic progenitor cells in syngeneic mice led in >90% of the recipient animals to development of a form of leukemia indistinguishable from its human counterpart. Retinoic acid treatment in vivo (through retinoic acid pellets implanted subcutaneously) leads to a strong extension of survival of leukemic mice, triggering (as in human acute promyelocytic leukemia) terminal differentiation of leukemic blasts (Figure 13 and not shown). VPA treatment (through I.P. injections of 400 mg/kg VPA every 12 hours) also significantly extended survival of leukemic mice (Figure 13).
Most strikingly, the combination of retinoic acid and VPA showed the greatest extension of survival and no leukemic blasts were observed in the peripheral blood and in the internal organs examined (bone marrow, spleen), for the entire duration of the treatment (Figure 13). This impressive result of the combination of retinoic acid and VPA in an in vivo model of leukemia shows that VPA may be administered in combination with a differentiating agent (such as retinoic acid) to induce an at least additive - but more likely synergistic - therapeutic biological response in the treatment of leukemias.

### Methods:

Mice that developed leukemia after inoculation with PML-RAR expressing cells were sacrificed. Single-cell suspensions of spleenocytes were prepared, and secondary recipient mice were reinoculated with 10⁷ cells. When leukemic blasts were evident in peripheral blood, and internal organs (bone marrow, spleen) were already macroscopically invaded by the leukemic cells, treatment was started by I.P. injection of VPA (400 mg/kg) every twelve hours, and by subcutaneous implant of a slow-release pellet of retinoic acid. VPA treatment followed the schedule: 5 days x 2 times, 2 days interval, for three consecutive weeks.

In Figure 13 cumulative survival ("Cum. Survival") curves of leukemic mice left untreated (control), or treated with VPA, RA, or RA + VPA (see Methods) are presented. The numbers in parentheses indicate the number of mice in the representative experiment shown. The experiment was repeated twice with similar results.

### Example 13

VPA synergizes in combination with several chemotherapeutic drugs in inducing apoptosis of HCT-116 colon cancer cells.

HCT-116 cells were treated with 5-Fluoruracile (5-FU), a drug currently in use for the treatment of colon cancer patients. High concentrations of 5-FU were able to efficiently induce apoptosis of HCT-116 cells (not shown). At lower doses however, only a modest effect was observed, and the main biological response observed was a reduction in cell number due to reduction in cycling cells (Figure 14, see also figure 16). We used the lower doses in combinations with VPA. VPA treatment alone (up to one week) did not induce apoptosis in these particular cells, and only minimally affected cell number (Figure 15A-B and data not shown). Treatment with the combination of 5-FU and VPA resulted in a strong synergistic reduction in cell growth, and much higher levels of apoptosis than observed with 5-FU alone (Figure 16A-B).

To further characterize the mechanisms underlying the combinatorial VPA + chemotherapeutic drug synergy, we investigated whether VPA pre-treatment was sufficient to achieve a similar response. The following drugs were used: 5-FU, adriamicine (AD), and irinothecan (IT). At the concentrations used, these drugs did not induce apoptosis significantly, and only moderately affected the growth rate of HCT-116 cells (Figure 14). Cells were pre-treated with VPA for three days, and then treated simultaneously with VPA + AD, VPA + FU or VPA + IT for up to 48 hours (Figure 17A-C). Strikingly, VPA pre-treatment resulted in a dramatic synergistic enhancement of apoptosis in cells exposed to any of the chemotherapeutic agents (Figure 17A-C). In parallel experiments, removal of VPA (24 hours wash-out following a 3-days pre-treatment) resulted in the lack of sensitization, showing that VPA must be administered concomitantly with the chemotherapeutic drug to achieve its sensitizing effect (Figure 17A-C). Taken together, these results show that the use of VPA leads to sensitization of tumor cells to the effect of several classes of drugs with anti-tumor activity and may result in synergistic activity of such combinatorial therapeutic treatments.

### Methods:

For Figure 14 cells were seeded in 6 well culture dishes at 100000 cells/ well. The day after, they were treated with the indicated drugs (5-FU: 2µM 5-Fluoruracile; AD: 20ng/ml adriamycin; IT: 3µM irinothecan), and then cultured for further 48 hours. Cells were counted at 24 h and 48 h after treatment. All of the assays have been performed in triplicate.

For Figure 15 cells were seeded in 6 well culture dishes at 100000 cells/ well. The day after, they were treated with the indicated concentrations of VPA, and then cultured for 48 hours. (A), results of triplicate counts of viable cells. (B), cells were stained with propidium iodide after permeabilization and fixation. Results of a cell cycle analysis are presented as percentage (%) of cells in G1, S, G2+M, and sub-G1 (apoptotic cells): all of the assays have been performed in triplicate.

For Figure 16 cells were seeded in 6 well culture dishes at 100000 cells/ well. The day after, they were treated for 72 hours in the presence of the indicated concentrations of 5-Fluoruracile (5-FU, 2µM), or 5-FU in combination with VPA (1mM). (A), results of triplicate counts of viable cells. (B), cells were stained with propidium iodide after permeabilization and fixation. Results of a cell cycle analysis are presented as percentage (%) of apoptotic cells: all of the assays have been performed in triplicate.

For Figure 17 cells were seeded in 10 cm culture dishes (1 million cells/dish). The day after, they were treated for 72 hours in the presence of VPA at a final concentration of 1mM. Cells were then seeded in 6 well culture dishes as described for figure 14, in the presence (+ / + series) or in the absence (+ / - series) of VPA. The day after, they were treated with the following drugs: 5-FU (panel A), AD (B), or IT (C), at the concentrations as indicated for figure 14. Cells were analyzed at the day of treatment, 24h, and 48h following treatment. Upper panels, results of triplicate counts of viable cells. Lower panels, cells were stained with propidium iodide after permeabilization and fixation. Results of a cell cycle analysis are presented as percentage (%) of apoptotic cells: all of the assays have been performed in triplicate.

### Example 14

Reduction in total cellular biomass of breast and prostate cancer cells upon treatment with VPA or the differentiation-inducing drug 1α,25 Dihydroxyvitamin D₃ alone, and upon combination of VPA and 1α,25 Dihydroxyvitamin D₃ (Figures 18-19).

Results using MCF-7 estrogen-dependent breast cancer cells are presented in figure 18 and using DU-145 prostate cancer cells in figure 19. Cells were cultured for 48 hours in the absence or presence of the indicated concentrations of 1α,25 Dihydroxyvitamin D₃ alone (Figure 18A and Figure 19A), in the absence or presence of the indicated concentrations of VPA alone or in combination of VPA at the indicated concentrations with 100 nM 1α,25 Dihydroxyvitamin D₃ (Figure 18B and Figure 19B). The effect on cell growth was measured by SRB-assay as described in example 1.

A clear at least additive reduction in cellular biomass was observed upon combinatorial treatment with VPA and the differentiation drug 1α,25 Dihydroxyvitamin D₃ used at the same time, compared to treatment with VPA or 1α,25 Dihydroxyvitamin D₃ alone in breast and prostate cancer cells. In MCF-7 cells a slight synergistic effect of the combination treatment could be observed.

### Example 15

VPA enhances the therapeutic effect of retinoic acid (RA) in the treatment of acute myeloid leukemia (AML) blast cells (Figures 20-26) in an additive and/or synergistic fashion.

We tested the effect of VPA as single agent or in combination with retinoic acid (RA) on acute myeloid leukemia (AML) blasts such as the Kasumi-1 cell line (Figure 20), that contains the t(8;21) and expresses the AML1/ETO fusion protein and fresh blasts from the bone marrow or peripheral blood of informed AML patients showing an initial percentage of circulating blasts greater than 70% (Figure 22). Cases were classified according to the French-American-British (FAB) classification (Bennett et al., Ann Intern Med *103*, 1985). Cytogenetic analysis and RT-PCR tests were performed to rule out the presence of the APL associated fusion genes according to standard methods as described (Mandelli et al., Blood 90, 1997; Mancini et al., Br J Haematol. 91, 1995; Grimwade et al., Blood 90, 1999). Results :
In Kasumi-1 cells (Figure 20) and in AML blasts (Figure 22) belonging to the M0, M2 and M4 FAB subtypes, we found that VPA as a single agent induced a partial myeloid differentiation, and in combination with RA triggered a complete and thus synergistic myeloid differentiation revealed by the appearance of cells with metamyelocyte- or neutrophil-like morphology and by the increased number of positive cells in the NBT dye reduction assay (up to 65%) (see Figures 20 and 21 for Kasumi-1 cells, Figure 22 and 24 for AML blasts). In addition, in Kasumi-1 cells a cell cycle analysis revealed an enhanced cell cycle shift into G1 arrest upon treatment using the combination of VPA plus RA compared to VPA or RA treatment alone (Figure 23). Most intriguingly, in AML blasts, treatment with VPA plus RA affected myeloid differentiation independently from the presence of a specific genetic lesion (Figure 22).

Interestingly, in combination with RA, VPA induced myeloid differentiation of blasts from either primary or relapsed AMLs (3 and 2 cases studied, respectively). In addition VPA, but not TSA, was found effective in inducing apoptosis of AML blasts as evaluated by FACS analysis of propidium-iodide stained cells (4 cases tested) which further indicates the therapeutic advantages which may be achieved using the HDAC inhibitory activity of VPA.

Furthermore, this induction of apoptosis was strongly enhanced, often in a synergistic fashion, upon treatment using the combination of VPA plus RA compared to VPA or RA treatment alone (Figure 25 and 26) and was also evident by cell cycle analysis as presented in Figure 27 by the appearance of an increased sub-G1-peak, representing apoptotic cells.

### Example 16

Anti-angiogenesis: Initially the effect of VPA was tested on human endothelial cells alone. In addition, the effect of inhibitors of vascular endothelial growth factor receptor (VEGF-R) tyrosine kinase activity was tested on these cells since VEGF-R inhibitors are known to act anti-angiogenic in model systems of tumor-associated endothelial cell activation (e.g. vascular tube formation and/or endothelial cell activation). Finally, the differentiation and/or apoptosis-inducing activity of VPA was tested in combination with VEGF-R tyrosine kinase inhibitors on the potential of endothelial cell activation, thus on the ability of these cells to initiate processes required for vasculargenesis.

The combinatorial treatment of endothelial cells using VPA and inhibitors of VEGF-R tyrosine kinase activity at the same time had an at least additive effect on the activation of these endothelial cells compared to the use of the individual drugs alone. Thus, VPA may be used in combination with inhibitors of angiogenic processes to achieve an enhanced therapeutic effect in respect to the inhibition of tumor vascularization via inhibition of the tumor-induced activation of endothelial cells.

## Claims

1. The use of a compound of formula I 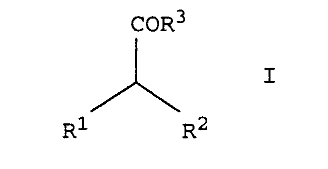 wherein R¹ and R² independently are a linear or branched, saturated or unsaturated, aliphatic C₃₋₂₅ hydrocarbon chain which optionally comprises one or several heteroatoms and which may be substituted, R³ is hydroxyl, halogen, alkoxy or an optionally alkylated amino group, or of pharmaceutically acceptable salts thereof for the manufacture of a medicament to sensitize human cancer cells for treatment efficacy in combination therapy with clinically established anti-cancer therapeutic drugs.

2. A use according to claim 1, wherein R¹ and R² independently are a linear or branched C₃₋₂₅ hydrocarbon chain which optionally comprises one double or triple bond.

3. A use according to claim 1 or 2, wherein the compound is selected from the group consisting of VPA, 4-yn VPA, and pharmaceutically acceptable salts thereof.

4. A use according to anyone of claims 1 to 3 , wherein the combination therapy using the sensitizing agent of formula I comprises treatment with differentiation inducing drugs, treatment with chemotherapeutic drugs, treatment with cytotoxic drugs, hormone therapy, immunotherapy, anti-angiogenic therapy and/or gene therapy.

5. A use according to anyone of claims 1 to 4 wherein the combination therapy using the sensitizing agent of formula I includes treatment with differentiation inducing drugs.

6. A use according to claim 5 wherein the differentiation inducing drug is selected from the group consisting of vitamin A based drugs, vitamin D₃ based drugs, and cytokines.

7. A use according to anyone of claims 1 to 4 wherein the combination therapy using the sensitizing agent of formula I includes immunotherapy.

8. A use according to claim 7 wherein the immunotherapy includes the use of an antibody.

9. A use according to claim 8 wherein the antibody is conjugated with a functional group, such as a cytotoxic protein or drug component.

10. A use according to claim 8 wherein the antibody is conjugated with a radioisotope.

11. A use according to claim 7 where the immunotherapy includes tumor vaccination.

12. A use according to anyone of claims 1 to 4 wherein the combination therapy using the sensitizing agent of formula I includes treatment with an antagonistic acting hormonal reagent.

13. A use according to anyone of claims 1 to 12 wherein the combination therapy using the sensitizing agent of formula I includes at least two methods of anti-tumor therapy as defined in claim 4.

14. A use according to anyone of claims 1 to 13, wherein the human cancer is selected from the group consisting of minimal residual tumor disease, tumor metastasis, skin cancer, estrogen receptor-dependent and independent breast cancer, ovarian cancer, prostate cancer, renal cancer, colon and colorectal cancer, pancreatic cancer, head and neck cancer, small cell and non-small cell lung carcinoma, and cancer of blood cells.

15. A use according to anyone of claims 1 to 14 wherein the sensitizing agent of formula I is an inhibitor of enzymes having HDAC activity and causes an additive therapeutic effect upon combinatorial therapy with one or several other anti-cancer treatments.

16. A use according to claim 15 wherein the enzyme having histone deacetylase activity is a mammalian, preferably a human histone deacetylase.

17. A use according to claim 15 or 16, wherein the human histone deacetylase is selected from the group consisting of HDACs 1-8 and members of the SIR2 protein family.

18. A use according to anyone of claims 15 to 17 wherein the sensitizing agent of formula I specifically inhibits only a subset of HDACs.

19. A use according to anyone of claims 1 to 18 wherein the sensitizing agent of formula I is used for the induction of differentiation of cells.

20. A use according to anyone of claims 1 to 18 wherein the sensitizing agent of formula I is used for the induction of differentiation of transformed cells.

21. A use according to anyone of claims 1 to 18 wherein the sensitizing agent of formula I is used for the induction of apoptosis of transformed cells.

22. A use according to anyone of claims 1 to 21, wherein the induction of hyperacetylation of histones or other proteins functionally regulated by acetylation has a beneficial effect for the treatment of human cancer.

23. A use according to anyone of claims 1 to 22, wherein the sensitizing agent of formula I or a pharmaceutically acceptable salt thereof is administered as a first therapeutic agent, and an anti-cancer agent is administered as a second therapeutic agent, **characterized in that** the daily dosage of said anti-cancer agent is significantly reduced compared to the daily dosage of the anti-cancer agent when administered alone.

24. A method for reducing the dosage of an anti-cancer agent comprising administering to a cancer patient an amount of a compound of formula I or a pharmaceutically acceptable salt thereof as defined in claims 1 to 3 effective to sensitize cancer cells in the patient.

25. A method of treating cancer in a patient which comprises administering to the patient an amount of a compound of formula I or a pharmaceutically acceptable salt thereof as defined in claims 1 to 3 effective to sensitize the cancer cells in the patient to an anti-cancer agent and a therapeutically effective amount of the anti-cancer agent.

26. A method of enhancing the therapeutic activity of an anti-cancer agent which comprises administering to a patient an amount of a compound of formula I or a pharmaceutically acceptable salt thereof as defined in claims 1 to 3 effective to sensitize cancer cells in the patient to the anti-cancer agent.

27. A pharmaceutical kit comprising as a first therapeutic agent a compound of formula I as defined in claim 1 or a pharmaceutically acceptable salt thereof, and as a second therapeutic agent an anti-cancer agent, wherein the anti-cancer agent is provided in a form suitable for administration in a daily dosage which is significantly reduced compared to the dosage of the anti-cancer agent when administered alone.

28. The use of a substance which is metabolized in patients to a sensitizing agent as defined in claims 1 to 3, for the manufacture of a medicament for a combinatorial treatment of human cancer.

29. A method for the identification of sensitizing agents being useful for combinatorial cancer therapy which comprises
providing a derivative of valproic acid,
determining its histone deacetylase inhibitory activity,
determining its efficiency in combinatorial cancer therapy, and
selecting the substance if the substance has histone deacetylase inhibitory activity and an efficiency in combinatorial cancer therapy which is significantly higher than that of the respective treatments alone.

30. A method according to claim 29 wherein the histone deacetylase inhibitory activity is determined by
a transcription repression assay,
a Western Blot detecting acetylation of histone H3 or histone H4, or
an enzymatic deacetylase assay.

31. A method according to claim 29 or 30 wherein the combinatorial therapeutic effect is measured in cell culture or by using in vivo animal tumor models.

32. A method according to claim 31 including a method which comprises cell cycle analysis, detecting apoptotic cells, measuring the number of viable cells or tumor size, detection of cellular differentiation markers, determining the metabolic activity of cells, and/or determining cell membrane integrity.

33. A method for the identification of genes regulated by combinatorial treatment with valproic acid or a derivative thereof and one or several other methods of anti-tumor therapy which comprises
a) providing two populations of cells which are substantially identical;
b) contacting the first population with VPA or a derivative thereof;
c) subjecting the first population to treatment with one or several other methods of anti-tumor therapy; and
d) detecting genes or gene products which are expressed in the first population which had been contacted with VPA or a derivative thereof and were subjected to treatment with one or several other methods of anti-tumor therapy at a level significantly higher than in the second population which had not been contacted with VPA or a derivative thereof
wherein steps b) and c) can be carried out simultaneously or in any order.

34. A method according to claim 33, wherein substractive hybridization or screening of arrays of cDNA samples, expressed sequence tags or unigene collections is employed.

35. A method according to claim 33 or 34 comprising the use of nucleic acid technology.

36. A method according to claim 35, wherein hybridization or polymerase chain reaction is used for detection.

37. A method according to claim 33 or 34 comprising the use of specific antibodies against differentially regulated proteins for detection.

38. A method for the diagnosis of tumors comprising determining outside of the human or animal body the expression level of a gene identified according to a method according to anyone of claims 33 to 37.

39. A diagnostic method to identify tumors or tumor cells comprising the step of testing in vitro whether a tumor or tumor cells is/are responsive to combinatorial treatment with VPA or a derivative thereof and one or several other methods of anti-tumor therapy.

40. A diagnostic method according to claim 39 comprising a method according to anyone of claims 32 to 36.
